# EUROPÄISCHE PATENTANMELDUNG

(11) **EP 3 988 020 A1**
(43) Veröffentlichungstag der Anmeldung: **27.04.2022**
(21) Anmeldenummer: 21202446.7
(22) Anmeldetag: 13.10.2021
(51) Int. Cl.: A61B 5/103, A61B 5/22, A61B 5/00, A63B 6/02, A63B 24/00

(54) **VERFAHREN UND VORRICHTUNG ZUR KRAFTMESSUNG**

(30) Priorität: 20.10.2020 EP 20202759
(71) Anmelder: Kistler Holding AG, 8408 Winterthur (CH)
(72) Erfinder: Buechli, Katharina, 5076 Bözen (CH); Jenni, Dominik, 8004 Zürich (CH); Roetenberg, Daniel, 8307 Ottikon bei Kemptthal (CH); Panjan, Andrej, 8332 Gradac (SI); Sarabon, Nejc, 4000 Kranj (SI)

(57) **Zusammenfassung**

Die Erfindung betrifft ein Verfahren zur Kraftmessung von mindestens einem Athleten (A), wobei in einem ersten Schritt (I) Übungsdaten (D5) für Sprungübungen erzeugt werden, unter Verwendung einer Datenverarbeitungsanlage (4), von welcher die Übungsdaten (D5) an eine erste Rechnereinrichtung (5) gesendet werden; wobei in einem zweiten Schritt (II) Sprungübungen angeleitet werden, unter Verwendung der ersten Rechnereinrichtung (5) durch den Athleten (A), auf welcher die Übungsdaten (D5) an den Athleten (A) ausgegeben werden; welcher Athlet (A) Sprungübungen auf der Messplattform (1) ausführt; von welcher eine Sprungkraft der Sprungübungen gemessen wird, von welcher für die gemessene Sprungkraft Messdaten (D1) erzeugt werden, und von welcher die Messdaten (D1) an die Datenverarbeitungsanlage (4) gesendet werden; wobei in einem dritten Schritt (III) die Messdaten (D1) ausgewertet werden, unter Verwendung der Datenverarbeitungsanlage (4), auf welcher die Messdaten (D1) zu Leistungsdaten (D10) ausgewertet werden, auf welcher für die Leistungsdaten (D10) Expertendaten (D4) ermittelt werden, und von welcher die Leistungsdaten (D10) und die Expertendaten (D4) an mindestens eine der Folgenden, der ersten Rechnereinrichtung (5) und einer zweiten Rechnereinrichtung (6) gesendet werden; und wobei in einem vierten Schritt (IV) die Leistungsdaten (D10) und die Expertendaten (D4) auf mindestens einer der Folgenden, der ersten Rechnereinrichtung (5) und der zweiten Rechnereinrichtung (6) ausgegeben werden.

## Beschreibung

### Technisches Gebiet

Die Erfindung betrifft ein Verfahren und eine Vorrichtung zur Kraftmessung nach dem Oberbegriff des unabhängigen Anspruchs.

### Stand der Technik

In der Schrift DE10040623A1 wird ein Verfahren zur Sprungkraftmessung eines Athleten offenbart. Dazu steigt der Athlet auf eine Messplattform mit mehreren Kraftsensoren. Der Athlet führt auf der Messplattform Übungen mit vertikalen Sprüngen aus. Die Messplattform misst für jeden Sprung eine vertikale Sprungkraft des Athleten und erzeugt dazu Messdaten. Die Messdaten werden einer Datenverarbeitungsanlage zugeführt und dort ausgewertet. Das Ergebnis der Auswertung wird Leistungsdiagnostik genannt. Die Leistungsdiagnostik umfasst Leistungsinformation zur Höhe der Sprünge des Athleten, zur Geschwindigkeit des Athleten bei den Sprüngen, zur Kraft des Athleten bei den Sprüngen, usw.

Eine solche Leistungsdiagnostik hat im Sport und in der Medizin eine erhebliche Bedeutung. So gibt sie Auskunft über einen Leistungszustand des Athleten. Der Leistungszustand besagt, wie gut Eigenschaften wie Kraft, Ausdauer, Schnelligkeit, Koordination und Beweglichkeit beim Athleten entwickelt sind.

Typischerweise dauert die Sprungkraftmessung des Athleten rund 6min. Der Athlet führt Übungen mit Einzelsprüngen oder Mehrfachsprüngen aus. Bei einem Einzelsprung erfolgt ein einzelner Sprung, bei Mehrfachsprüngen findet innerhalb eines Zeitintervalls eine Abfolge von Sprüngen als Dreiersprünge, Fünfersprünge, usw. statt. Die Sprungübungen werden vom Athleten mehrmals wiederholt. Da bei diesem Verfahren immer nur ein Athlet die Messplattform benutzen kann, müssen andere Athleten in der Zwischenzeit warten. Die Sprungkraftmessung einer Mannschaft mit zwanzig Athleten dauert dann 2:00h. Um eine Wartezeit kurz zu halten, müssen sich die Athleten für die Sprungkraftmessung genau an einen Zeitplan halten, was Stress verursacht. Es ist ein Bedürfnis der Athleten, die Sprungkraftmessung stressfrei auszuführen.

Die Leistungsdiagnostik richtet sich nicht nur an den Athleten, sondern auch an seinen Betreuer. In der Regel ist der Betreuer bei der Messplattform, um die Sprungübungen anzuleiten. Bei einer Mannschaft mit zwanzig Athleten resultiert in einem Zeitaufwand von 2:00h für den Betreuer. Es ist ein Wunsch des Betreuers, den Zeitaufwand für die Anleitung der Sprungübungen zu reduzieren.

Die Leistungsdiagnostik wird auf einem Bildschirm der Datenverarbeitungsanlage ausgegeben. Die Messplattform und die Datenverarbeitungsanlage sind räumlich nebeneinander angeordnet. Die Athleten und der Betreuer müssen also nach den Sprungübungen bei der Datenverarbeitungsanlage bleiben, um von der Leistungsdiagnostik Kenntnis zu erlangen. Dies ist mit weiterem Zeitaufwand verbunden. Auch hier möchten die Athleten und der Betreuer nicht so lange warten, um von der Leistungsdiagnostik Kenntnis zu erlangen.

Die Leistungsdiagnostik umfasst auch historische Leistungsinformation des Athleten. Die Athleten und der Betreuer werden somit über eine zeitliche Entwicklung des Leistungszustandes des Athleten informiert. Gerade im Leistungssport ist es das erklärte Ziel der Athleten und des Betreuers, den Leistungszustand des Athleten zu optimieren. In der Regel sind die Sprungübungen so gestaltet, dass der Athlet auf einen bestimmten Termin hin, einen optimalen Leistungszustand hat. Zur zielführenden Gestaltung der Sprungübungen sind die Athleten und der Betreuer auch an einer Interpretation der Leistungsdiagnostik interessiert.

Es ist daher eine erste Aufgabe der vorliegenden Erfindung, ein Verfahren und eine Vorrichtung bereitzustellen, mit denen die Athleten die Sprungkraftmessung stressfrei ausführen können. Eine zweite Aufgabe der Erfindung besteht darin, ein Verfahren und eine Vorrichtung anzugeben, welche den Zeitaufwand des Betreuers für die Anleitung der Sprungübungen reduziert. Und eine dritte Aufgabe der Erfindung ist auf eine rasche Kenntnisnahme der Leistungsdiagnostik durch die Athleten und den Betreuer gerichtet. Schliesslich stellt sich die Erfindung die vierte Aufgabe, ein Verfahren und eine Vorrichtung aufzuzeigen, welche die Athleten und den Betreuer bei der Interpretation der Leistungsdiagnostik unterstützt.

### Darstellung der Erfindung

Zumindest eine dieser Aufgaben wird durch die Merkmale des unabhängigen Anspruchs 1 gelöst.

Die Erfindung betrifft ein Verfahren zur Kraftmessung von mindestens einem Athleten, unter Verwendung einer Messplattform; wobei in einem ersten Schritt Übungsdaten für Sprungübungen erzeugt werden, unter Verwendung einer Datenverarbeitungsanlage, von welcher die Übungsdaten an eine erste Rechnereinrichtung gesendet werden; wobei in einem zweiten Schritt Sprungübungen angeleitet werden, unter Verwendung der ersten Rechnereinrichtung durch den Athleten, auf welcher die Übungsdaten an den Athleten ausgegeben werden; welcher Athlet Sprungübungen auf der Messplattform ausführt; von welcher eine Sprungkraft der Sprungübungen gemessen wird, von welcher für die gemessene Sprungkraft Messdaten erzeugt werden, und von welcher die Messdaten an die Datenverarbeitungsanlage gesendet werden; wobei in einem dritten Schritt die Messdaten ausgewertet werden, unter Verwendung der Datenverarbeitungsanlage, auf welcher die Messdaten zu Leistungsdaten ausgewertet werden, auf welcher für die Leistungsdaten Expertendaten ermittelt werden, und von welcher die Leistungsdaten und die Expertendaten an mindestens eine der Folgenden, der ersten Rechnereinrichtung und einer zweiten Rechnereinrichtung gesendet werden; und wobei in einem vierten Schritt die Leistungsdaten und die Expertendaten auf mindestens einer der Folgenden, der ersten Rechnereinrichtung und der zweiten Rechnereinrichtung ausgegeben werden.

Der Vorteil der Erfindung besteht darin, dass der Betreuer die Sprungübungen nicht mehr selbst anleiten muss, was dessen Zeitaufwand reduziert. Zudem erhält der Athlet an der ersten Rechnereinrichtung und/oder der Betreuer an der zweiten Rechnereinrichtung die ausgewerteten Leistungsdaten zeitnah zugestellt. Und dann werden für die Leistungsdaten auch Expertendaten ermittelt, welche Expertendaten dem Athleten und/oder dem Betreuer bei der Interpretation der Leistungsdaten unterstützen. Die Effizienz der Sprungübungen des Athleten wird dadurch gezielt verbessert.

Die Erfindung betrifft auch eine Vorrichtung zur Durchführung des Verfahrens, mit einer Messplattform, einer Datenverarbeitungsanlage und einer ersten Rechnereinrichtung, wobei die Vorrichtung auch eine Datenübertragungsvorrichtung aufweist; wobei die Datenverarbeitungsanlage über die Datenübertragungsvorrichtung Übungsdaten für Sprungübungen an die erste Rechnereinrichtung sendet; wobei die Messplattform einen Messplattform-Prozessor aufweist, der derart konfiguriert ist, dass er für auf der Messplattform ausgeführte Sprungübungen Messdaten erzeugt; wobei die Messplattform über die Datenübertragungsvorrichtung die Messdaten an die Datenverarbeitungsanlage sendet; wobei die Datenverarbeitungsanlage einen Haupt-Prozessor aufweist, der derart konfiguriert ist, dass er die Messdaten zu Leistungsdaten auswertet und dass er für die Leistungsdaten Expertendaten ermittelt; und wobei die Datenübertragungsvorrichtung über die Datenübertragungsvorrichtung die Leistungsdaten und die Expertendaten an mindestens eine der Folgenden, der ersten Rechnereinrichtung und einer zweiten Rechnereinrichtung sendet.

### Kurze Beschreibung der Zeichnungen

Im Folgenden wird die Erfindung beispielhaft unter Beizug der Figuren näher erklärt. Es zeigen
- Fig. 1: ein Flussdiagramm mit Schritten I bis IV des Verfahrens;
- Fig. 2: eine schematische Darstellung einer Vorrichtung 10 zur Durchführung des Verfahrens nach Fig. 1;
- Fig. 3: ein Flussdiagramm mit ersten Unterschritten XI bis XIII eines ersten Schrittes I des Verfahrens nach Fig. 1;
- Fig. 4: eine schematische Darstellung einer Ausgabe von im ersten Schritt I des Verfahrens nach Fig. 3 bereitgestellten Übungsdaten D5 auf einem ersten Ausgabemittel AU5;
- Fig. 5: eine schematische Darstellung einer Ausgabe von im ersten Schritt I des Verfahrens nach Fig. 3 bereitgestellten Übungsdaten D5 auf einem zweiten Ausgabemittel AU6;
- Fig. 6: ein Flussdiagramm mit zweiten Unterschritten XXI bis XXIII eines zweiten Schrittes II des Verfahrens nach Fig. 1;
- Fig. 7: ein Flussdiagramm mit dritten Unterschritten XXXI bis XXXIV eines dritten Schrittes III des Verfahrens nach Fig. 1;
- Fig. 8: ein Flussdiagramm mit vierten Unterschritten XLI bis XLIII eines vierten Schrittes IV des Verfahrens nach Fig. 1;
- Fig. 9: eine schematische Darstellung einer Ausgabe von im dritten Schritt III des Verfahrens nach Fig. 7 ermittelten Leistungsdaten D10 und Expertendaten D4 auf dem ersten Ausgabemittel AU5;
- Fig. 10: eine schematische Darstellung einer Ausgabe von im dritten Schritt III des Verfahrens nach Fig. 7 ermittelten Leistungsdaten D10 und Expertendaten D4 auf dem zweiten Ausgabemittel AU6;
- Fig. 11: eine graphische Darstellung einer Ausgabe einer im dritten Schritt III des Verfahrens nach Fig. 7 ermittelten fünften Experteninformation E5;
- Fig. 12: eine schematische Darstellung einer Ausgabe von im ersten Schritt I des Verfahrens nach Fig. 3 bereitgestellten Übungsdaten D5 und von im dritten Schritt III des Verfahrens nach Fig. 7 ermittelten Leistungsdaten D10 und Expertendaten D4 auf dem ersten Ausgabemittel AU5; und
- Fig. 13: eine schematische Darstellung einer Ausgabe von im ersten Schritt I des Verfahrens nach Fig. 3 bereitgestellten Übungsdaten D5 auf einem zweiten Ausgabemittel AU6.

Gleiche Bezugszeichen bezeichnen in den Figuren gleiche Gegenstände.

### Wege zur Ausführung der Erfindung

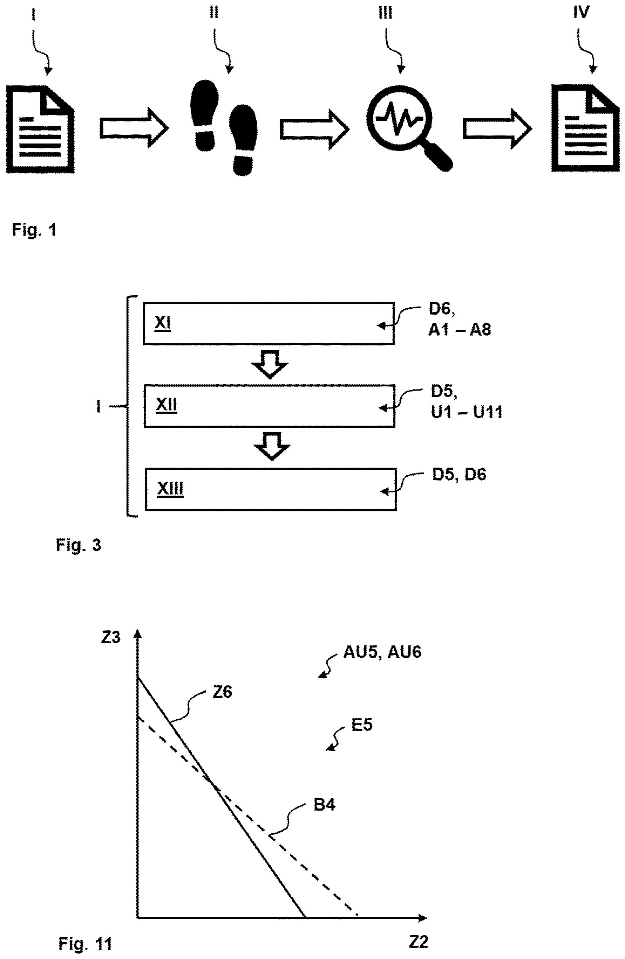

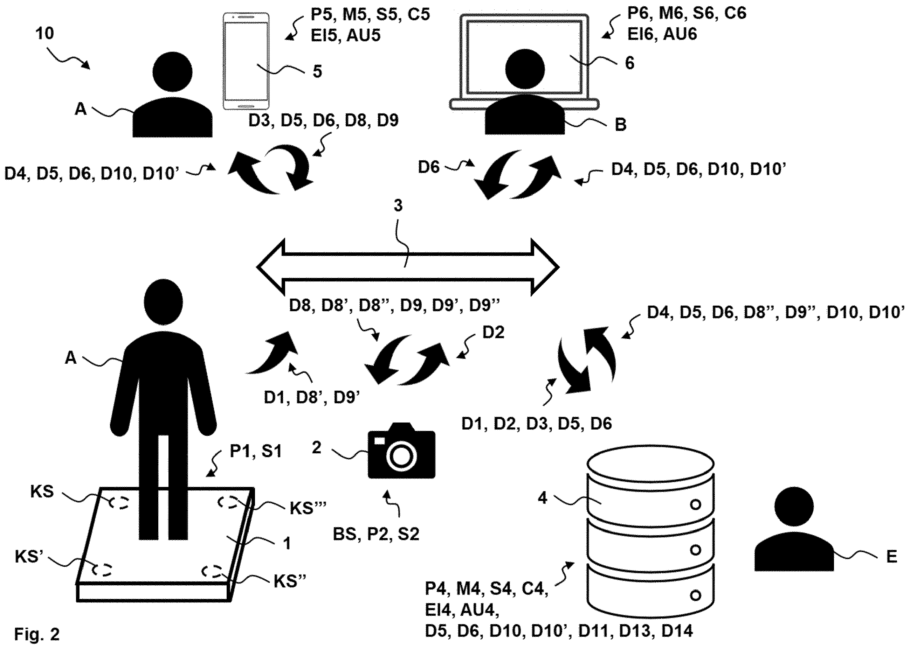

Fig. 1 zeigt ein Flussdiagramm mit einem ersten Schritt I des Verfahrens, mit einem zweiten Schritt II des Verfahrens, mit einem dritten Schritt III des Verfahrens und mit einem vierten Schritt IV des Verfahrens. Fig. 2 zeigt eine schematische Darstellung einer Vorrichtung 10 zur Durchführung des Verfahrens.

Im Sinne der vorliegenden Erfindung wird zwischen digitalen Daten und Informationen unterschieden. Digitale Daten sind an eine Maschine wie einen Prozessor, ein Computerprogramm, ein Speichermedium, usw. gerichtet, während Informationen an einen Menschen wie einen Athleten, einen Betreuer, usw. gerichtet sind. Digitale Daten sind nur für die Maschine verständlich und verarbeitbar, während Informationen nur für den Menschen verständlich und verarbeitbar sind.

Die Vorrichtung 10 weist mindestens eine Messplattform 1 auf. Die Messplattform 1 weist eine Standfläche von knapp 1m² auf. Zur Ausführung der Sprungübungen steigt der Athlet A auf die Standfläche. Die Messplattform 1 ist ein Diagnosegerät zur Ermittlung des Leistungszustandes des Athleten A. Die Messplattform 1 ist kein Trainingsgerät, um den Leistungszustand des Athleten A zu optimieren. Trainieren, um den Leistungszustand zu optimieren tut der Athlet A an bekannten Trainingsgeräten wie an Kraftmaschinen für das Trainieren der Kraft, auf Laufbändern und Fahrrädern für das Trainieren der Ausdauer und Schnelligkeit, usw. Die Messplattform 1 weist mehrere Kraftsensoren KS, KS', KS'', KS''' und mindestens einen Messplattform-Prozessor P1 auf. Für jeden vertikalen Sprung des Athleten A messen die Kraftsensoren KS, KS', KS'', KS''' eine Sprungkraft. Die Sprungkraft ist eine Bodenreaktionskraft. Nach dem dritten Newtonschen Gesetz ist die Bodenreaktionskraft diejenige Kraft, die der Boden auf den Athleten A ausübt, der mit dem Boden in Kontakt steht. In Ruheposition im Stand entspricht die Bodenreaktionskraft dem Gewicht des Athleten A. Bei Bewegungen verändert sich die Bodenreaktionskraft durch Beschleunigungskräfte. So wirkt beim Laufen ein Zwei- bis Dreifaches der Kraft, die dem Gewicht des Athleten A entspricht, auf den Athleten A ein. Ein Messbereich der Kraftsensoren KS, KS', KS'', KS''' erstreckt sich von 0 bis 10kN. Eine Messfrequenz der Kraftsensoren KS, KS', KS'', KS''' beträgt 500Hz. Der Messplattform-Prozessor P1 ist derart konfiguriert, dass er für die gemessene Sprungkraft Messdaten D1 erzeugt. Bei Kenntnis der vorliegenden Erfindung kann ein Athlet die Sprungübungen auch auf zwei Messplattformen gleichzeitig ausführen. Die Messplattformen sind identisch. Mit einem linken Bein kann der Athlet auf einer ersten Messplattform stehen und mit einem rechten Bein steht er auf einer zweiten Messplattform. Beide Messplattformen messen unabhängig voneinander eine Sprungkraft und erzeugen für die gemessene Sprungkraft Messdaten.

Die Vorrichtung 10 weist mindestens eine Kamera 2 auf. Die Kamera 2 weist mindestens einen Bildsensor BS und mindestens einen Kamera-Prozessor P2 auf. Der Bildsensor BS erfasst Bilder des Athleten A während der Ausführung der Sprungübungen. Eine Erfassungsfrequenz des Bildsensors BS beträgt 100fps. Der Kamera-Prozessor P2 ist derart konfiguriert, dass er für die erfassten Bilder Bilddaten D2 erzeugt.

Die Vorrichtung 10 weist mindestens eine Datenübertragungseinrichtung 3 zur Übertragung von digitalen Daten auf. Die digitalen Daten werden auf einem Übertragungsweg zwischen Schnittstellen übertragen. Die Schnittstellen sind derart konfiguriert, dass sie ein Senden und Empfangen von digitalen Daten ausführen. Die digitalen Daten sind Messdaten D1, Bilddaten D2, Expertendaten D4, Übungsdaten D5 und Athletendaten D6. Nach Fig. 2 sind digitale Daten als gebogene Pfeile dargestellt. Die Übertragung der digitalen Daten erfolgt gemäss einem Protokoll per Funk oder Kabel. Gemäss Protokoll sind die Schnittstellen über Identifikationsnummern eindeutig identifiziert. Die Datenübertragungseinrichtung 3 kann ein Universal Serial Bus (USB), Bluetooth, Ethernet, Internet, ein drahtloses lokales Netzwerk (WLAN), ein Festnetz, usw. sein. Die Messplattform 1 weist mindestens eine Messplattform-Schnittstelle S1 auf. Und auch die Kamera 2 weist mindestens eine Kamera-Schnittstelle S2 auf.

Die Vorrichtung 10 weist mindestens eine Datenverarbeitungsanlage 4 auf. Die Datenverarbeitungsanlage 4 wird von mindestens einem Entwickler E wie einen Techniker, usw. verwendet. Die Datenverarbeitungsanlage 4 kann an einem beliebigen Ort auf der Welt angeordnet sein. Die Datenverarbeitungsanlage 4 weist mindestens einen Haupt-Prozessor P4, mindestens ein Haupt-Speichermedium M4 für digitale Daten und mindestens eine Haupt-Schnittstelle S4 zum Senden und Empfangen von digitalen Daten auf. Mindestens ein Haupt-Computerprogramm C4 ist in den Prozessor P4 geladen und wird vom Haupt-Prozessor P4 ausgeführt. Das ausgeführte Haupt-Computerprogramm C4 veranlasst den Haupt-Prozessor P4 den ersten Schritt I des Verfahrens und den dritten Schritt III des Verfahrens auszuführen. Die Datenverarbeitungsanlage 4 weist mindestens ein Haupt-Eingabemittel EI4 wie eine Tastatur, ein Mikrofon, eine Kamera, usw. auf. Die Datenverarbeitungsanlage 4 weist mindestens ein Haupt-Ausgabemittel AU4 wie einen Bildschirm, einen Lautsprecher, usw. auf.

Die Vorrichtung 10 weist mindestens eine erste Rechnereinrichtung 5 und mindestens eine zweite Rechnereinrichtung 6 auf. Die erste Rechnereinrichtung 5 wird vom Athleten A verwendet. Der Athlet A hat mindestens einen Betreuer B wie einen Trainer, einen Therapeuten, usw. Die zweite Rechnereinrichtung 6 wird vom Betreuer B verwendet. Vorzugsweise werden die erste und zweite Rechnereinrichtung 5, 6 am Ort der Kraftmessung benutzt. Die erste und zweite Rechnereinrichtung 5, 6 können ein Personal Computer (PC), ein Laptop, ein Smartphone, eine Smartwatch, usw. sein. Im Beispiel nach Fig. 2 ist die erste Rechnereinrichtung 5 ein Smartphone, während die zweite Rechnereinrichtung 6 ein PC ist.

Die erste Rechnereinrichtung 5 weist mindestens einen ersten Prozessor P5, mindestens ein erstes Speichermedium M5 für digitale Daten und mindestens eine erste Schnittstelle S5 zum Senden und zum Empfangen von digitalen Daten auf. Mindestens ein erstes Computerprogramm C5 ist in den ersten Prozessor P5 geladen und wird vom ersten Prozessor P5 ausgeführt. Das ausgeführte erste Computerprogramm C5 veranlasst den ersten Prozessor P5 den zweiten Schritt II des Verfahrens und den vierten Schritt IV des Verfahrens auszuführen. Die erste Rechnereinrichtung 5 weist mindestens ein erstes Eingabemittel EI5 wie eine Taste, ein Berührungsbildschirm (Touchscreen), ein Mikrofon, eine Kamera, usw. auf. Die erste Rechnereinrichtung 5 weist mindestens ein erstes Ausgabemittel AU5 wie einen Berührungsbildschirm, einen Lautsprecher, usw. auf.

Die zweite Rechnereinrichtung 6 weist mindestens einen zweiten Prozessor P6, mindestens ein zweites Speichermedium M6 für digitale Daten und mindestens eine zweite Schnittstelle S6 zum Senden und zum Empfangen von digitalen Daten auf. Mindestens ein zweites Computerprogramm C6 ist in den zweiten Prozessor P6 geladen und wird vom zweiten Prozessor P6 ausgeführt. Das ausgeführte zweite Computerprogramm C6 veranlasst den zweiten Prozessor P6, den vierten Schritt IV des Verfahrens auszuführen. Die zweite Rechnereinrichtung 6 weist mindestens ein zweites Eingabemittel EI6 wie mindestens eine Tastatur, ein Mikrofon, eine Kamera, usw. auf. Die zweite Rechnereinrichtung 6 weist mindestens ein zweites Ausgabemittel AU6 wie einen Bildschirm, einen Lautsprecher, usw. auf.

### ERSTER SCHRITT I

Im ersten Schritt I werden Übungsdaten D5 für Sprungübungen erzeugt. Fig. 3 zeigt den ersten Schritt I im Detail, mit einem elften Unterschritt XI, einem zwölften Unterschritt XII und einem dreizehnten Unterschritt XIII.

Im elften Unterschritt XI wird mindestens eine Athleteninformation A1 bis A8 bereitgestellt. Mit der Athleteninformation A1 bis A8 ist jeder Athlet A eindeutig identifizierbar. Für einen neu vom Betreuer B betreuten Athleten A wird die Athleteninformation A1 bis A8 erstmals erzeugt. Die Athleteninformation A1 bis A8 ist eine alphanumerische Zeichenfolge, ein Bild, ein Graph, usw. Die Bereitstellung der Athleteninformation A1 bis A8 kann auf vielfältige Art und Weise erfolgen:
- Die Athleteninformation A1 bis A8 kann vom Betreuer B über das zweite Eingabemittel EI6 in die zweite Rechnereinrichtung 6 eingegeben werden und über die zweite Schnittstelle S6 als Athletendaten D6 an die Datenverarbeitungsanlage 4 gesendet werden. Die Athletendaten D6 können von der Datenverarbeitungsanlage 4 über die Haupt-Schnittstelle S4 empfangen und im Haupt-Speichermedium M4 gespeichert werden. Die Athleteninformation A2 bis A8 kann vom Athleten A auf diese Weise auch aktualisiert werden.
- Die Athleteninformation A1 bis A8 kann aber auch vom Athleten A über das erste Eingabemittel EI5 in die erste Rechnereinrichtung 5 eingegeben werden und über die erste Schnittstelle S5 als Athletendaten D6 an die Datenverarbeitungsanlage 4 gesendet werden. Die Athletendaten D6 können von der Datenverarbeitungsanlage 4 über die Haupt-Schnittstelle S4 empfangen und im Haupt-Speichermedium M4 gespeichert werden.
- Und die Athleteninformation A1 bis A8 kann vom Entwickler E über das Haupt-Eingabemittel EI4 in die Datenverarbeitungsanlage 4 eingegeben und im Haupt-Speichermedium M4 gespeichert werden.

Beispiele für die Athleteninformation A1 bis A8 sind:
- Eine erste Athleteninformation A1 ist eine Identifikationsnummer des Athleten A. Die erste Athleteninformation A1 wird einmal erzeugt und bleibt danach unverändert.
- Eine zweite Athleteninformation A2 ist ein Name des Athleten A.
- Eine dritte Athleteninformation A3 ist ein Geschlecht des Athleten A.
- Eine vierte Athleteninformation A4 ist ein Alter des Athleten A.
- Eine fünfte Athleteninformation A5 ist eine Grösse des Athleten A.
- Eine sechste Athleteninformation A6 ist ein Gewicht des Athleten A.
- Eine siebte Athleteninformation A7 ist eine Sportart des Athleten A.
- Eine achte Athleteninformation A8 ist eine frei bestimmbare Sporteigenschaft des Athleten A, wie "Stürmer", "Linksfüsser", "Junior", usw.

Im zwölften Unterschritt XII wird mindestens eine Übungsinformation U1 bis U11 erzeugt. Zur Erzeugung kann die Übungsinformation U1 bis U11 vom Entwickler E über das Haupt-Eingabemittel EI4 in die Datenverarbeitungsanlage 4 eingegeben und im Haupt-Speichermedium M4 als Übungsdaten D5 gespeichert werden. Die Übungsinformation U1 bis U11 wird einmal eingegeben und ist danach als Übungsdaten D5 aus dem Haupt-Speichermedium M4 beliebig oft abrufbar.

Die Übungsinformation U1 bis U11 umfasst Angaben zu Sprungübungen für Kraft, Ausdauer, Schnelligkeit, Koordination und Beweglichkeit des Athleten A. Mit der Übungsinformation U1 bis U11 weiss der Athlet A, welche Sprungübungen er wie viele Male, in welcher Reihenfolge und mit welcher Pausenzeit dazwischen er ausführen soll. Und auch der Betreuer B weiss mit den Übungsinformation U1 bis U11, welche Sprungübungen der Athlet A wie viele Male, in welcher Reihenfolge und mit welcher Pausenzeit dazwischen ausführen wird bzw. ausgeführt hat. Die Übungsinformation U1 bis U11 ist eine alphanumerische Zeichenfolge, ein Bild, usw.

Beispiele für die Übungsinformation U1 bis U11 sind:
- Eine erste Übungsinformation U1 bezeichnet das Übungsdatum.
- Eine zweite Übungsinformation U2 bezeichnet eine Reihenfolge der Sprungübungen.
- Eine dritte Übungsinformation U3 bezeichnet eine Pausenzeit zwischen den Sprungübungen.
- Eine vierte Übungsinformation U4 bezeichnet eine Anzahl von vertikalen Einzelsprüngen aus einer aufrechten Stellung heraus einbeinig links oder rechts oder beidbeinig (Countermovement Jump).
- Eine fünfte Übungsinformation U5 bezeichnet eine Anzahl von vertikalen Einzelsprüngen aus einer Hockstellung heraus einbeinig links oder rechts oder beidbeinig (Squat Jump).
- Eine sechste Übungsinformation U6 bezeichnet eine Anzahl von vertikalen Einzelsprüngen aus einer vordefinierten Fallhöhe heraus (Drop Jump).
- Eine siebte Übungsinformation U7 bezeichnet eine Anzahl von vertikalen Einzelsprüngen aus einer aufrechten Stellung heraus einbeinig links oder rechts oder beidbeinig (Countermovement Jump) mit einem vordefinierten Zusatzgewicht.
- Eine achte Übungsinformation U8 bezeichnet eine Anzahl von vertikalen Einzelsprüngen aus einer Hockstellung heraus einbeinig links oder rechts oder beidbeinig (Squat Jump) mit einem vordefinierten Zusatzgewicht.
- Eine neunte Übungsinformation U9 bezeichnet eine Zeitdauer von Mehrfachsprüngen.
- Eine zehnte Übungsinformation U10 bezeichnet eine Anzahl von Kniebeugen.
- Eine elfte Übungsinformation U11 bezeichnet eine Zeitdauer einer Gleichgewichtsstellung einbeinig links oder rechts oder beidbeinig mit optionalen Handicaps wie geschlossene Augen, Kopf im Nacken, usw.

Im dreizehnten Unterschritt XIII werden die Athletendaten D6 und die Übungsdaten D5 dem Athleten A und dem Betreuer B bereitgestellt. Dazu werden die Athletendaten D6 und die Übungsdaten D5 von der Datenverarbeitungsanlage 4 über die Haupt-Schnittstelle S4 an die erste Rechnereinrichtung 5 gesendet. Die erste Rechnereinrichtung 5 empfängt die Athletendaten D6 und die Übungsdaten D5 über die erste Schnittstelle S5. Die erste Rechnereinrichtung 5 speichert die Athletendaten D6 und die Übungsdaten D5 im ersten Speichermedium S5. Und die Athletendaten D6 und die Übungsdaten D5 werden von der Datenverarbeitungsanlage 4 über die Haupt-Schnittstelle S4 an die zweite Rechnereinrichtung 6 gesendet. Die zweite Rechnereinrichtung 6 empfängt die Athletendaten D6 und die Übungsdaten D5 über die zweite Schnittstelle S6. Die zweite Rechnereinrichtung 6 speichert die Athletendaten D6 und die Übungsdaten D5 im zweiten Speichermedium S6.

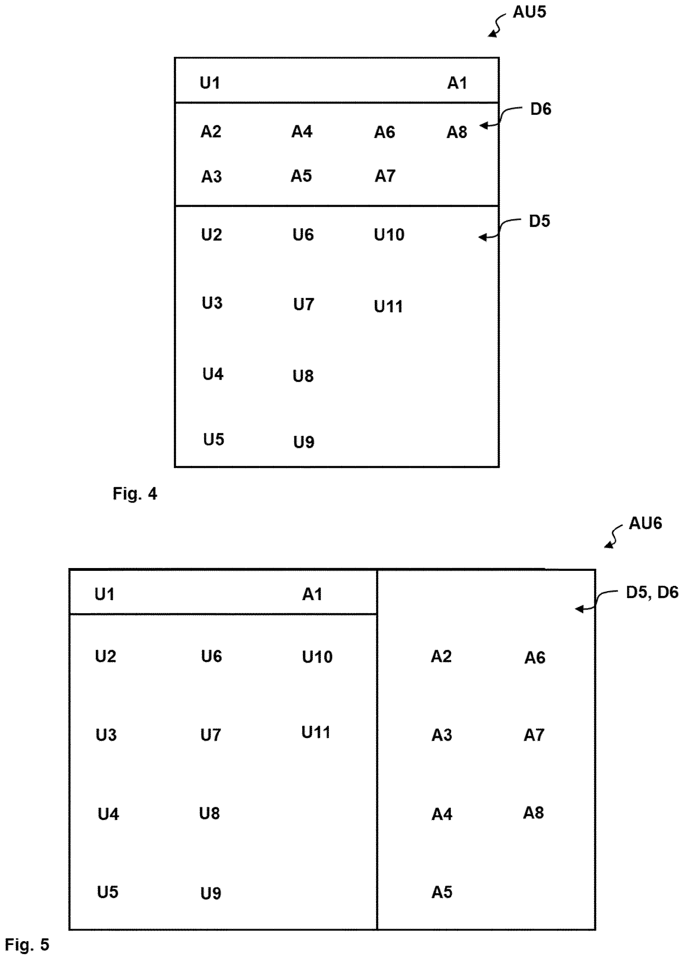

Fig. 4 zeigt eine schematische Darstellung der Athletendaten D6 und der Übungsdaten D5, welche vom ersten Computerprogramm C5 auf dem ersten Ausgabemittel AU5 der ersten Rechnereinrichtung 5 ausgegeben werden. Das erste Ausgabemittel AU5 ist beispielsweise ein Bildschirm mit 5.5'' Diagonale. Die Gesamtheit der Athletendaten D6 wird als Athleteninformation A1 bis A8 ausgegeben. Die Gesamtheit der Übungsdaten D5 wird als Übungsinformation U1 bis U11 ausgegeben. Der Athlet A erhält so in einer einzigen Ausgabe Kenntnis von relevanten Informationen. Der Athlet A kann auch den Zugriff auf das erste Ausgabemittel AU5 mit dem Betreuer B teilen, so dass der Athlet A und der Betreuer B gleichzeitig zeitnah Kenntnis von relevanten Informationen erhalten.

Fig. 5 ist eine schematische Darstellung der Athletendaten D6 und der Übungsdaten D5, welche vom zweiten Computerprogramm C5 auf dem zweiten Ausgabemittel AU6 der zweiten Rechnereinrichtung 6 ausgegeben werden. Das zweite Ausgabemittel AU6 ist beispielsweise ein Bildschirm mit 22'' Diagonale. Die Gesamtheit der Athletendaten D6 wird als Athleteninformation A1 bis A8 ausgegeben. Die Gesamtheit der Übungsdaten D5 wird als Übungsinformation U1 bis U11 ausgegeben. Der Betreuer B erhält so in einer einzigen Ausgabe Kenntnis von relevanten Informationen. Der Betreuer B kann auch den Zugriff auf das zweite Ausgabemittel AU6 mit dem Athleten A teilen, so dass der Betreuer B und der Athlet A gleichzeitig zeitnah Kenntnis von relevanten Informationen erhalten.

Der Betreuer B und der Athlet A kommunizieren über die Durchführung der Sprungübungen am Übungsdatum U1. Die Kommunikation kann über bekannte Kommunikationsmittel wie Telefon, Short Messaging Service (SMS), elektronische Post (E-Mail), usw. erfolgen. Das Ergebnis der Kommunikation ist eine Auswahl von Übungsdaten D5 mit mindestens einer Übungsinformation U1 bis U11 mit Angaben zu Sprungübungen, welche vom Athleten A am Übungsdatum U1 ausgeführt werden sollen.

### ZWEITER SCHRITT II

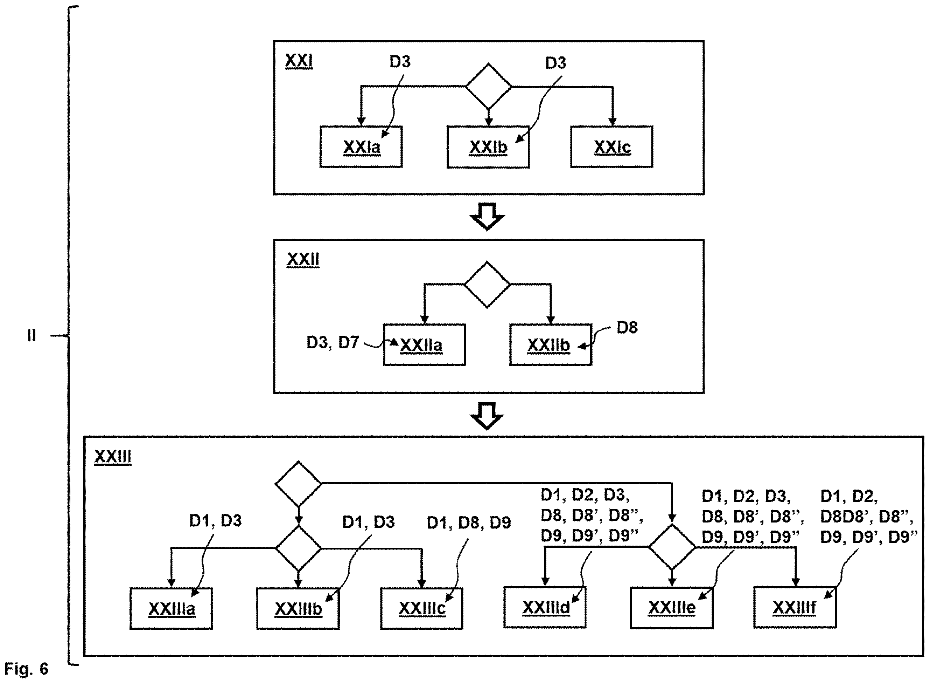

Im zweiten Schritt II erfolgt eine Anleitung von Sprungübungen. Es ist der Athlet A, der zu den Sprungübungen angeleitet wird. Im Beispiel nach Fig. 2 führt der Athlet A die Sprungübungen auf der Messplattform 1 aus. Fig. 6 zeigt den zweiten Schritt II im Detail mit einem einundzwanzigsten Unterschritt XXI, einem zweiundzwanzigsten Unterschritt XXII und einem dreiundzwanzigsten Unterschritt XXIII.

Der einundzwanzigste Unterschritt XXI weist drei Alternativen auf, mit einem ersten einundzwanzigsten Alternativschritt XXIa oder mit einem zweiten einundzwanzigsten Alternativschritt XXIb oder mit einem dritten einundzwanzigsten Alternativschritt XXIc.

Gemäss dem ersten einundzwanzigsten Alternativschritt XXIa begibt sich der Athlet A zur Messplattform 1. Die Messplattform 1 wird von der ersten Rechnereinrichtung 5 des Athleten A identifiziert. Beispielsweise weist die Messplattform 1 eine Identifikationsnummer wie einen Strich Code, einen Quick Response (QR) Code, usw. auf, mit der die Messplattform 1 eindeutig identifizierbar ist. Der Athlet A scannt die Identifikationsnummer mit dem ersten Eingabemittel EI5 in Form einer Kamera der ersten Rechnereinrichtung 5 ein. Das erste Computerprogramm C5 der ersten Rechnereinrichtung 5 erkennt die eingescannte Identifikationsnummer und erzeugt Identifikationsdaten D3 mit der erkannten Identifikationsnummer der Messplattform 1 und mit einer Identifikationsnummer der ersten Rechnereinrichtung 5, zur eindeutigen Identifizierung des Athleten A. Die erste Rechnereinrichtung 5 sendet die Identifikationsdaten D3 über die erste Schnittstelle S5 aus, welche von der Haupt-Schnittstelle S4 der Datenverarbeitungsanlage 4 empfangen werden. Das Haupt-Computerprogramm C4 der Datenverarbeitungsanlage 4 liest die Identifikationsdaten D3 ein.

Gemäss dem zweiten einundzwanzigsten Alternativschritt XXIb wird die erste Rechnereinrichtung 5 des Athleten A automatisch identifiziert, sobald sie in der Nähe der Messplattform 1 ist. Im Sinne der Erfindung bedeutet das Substantiv "Nähe" eine Entfernung von weniger als 30m. Beispielsweise sendet die erste Schnittstelle S5 der ersten Rechnereinrichtung 5 regelmässig Identifikationsdaten D3 mit einer Identifikationsnummer der ersten Rechnereinrichtung 5 aus, zur eindeutigen Identifizierung des Athleten A. Die Identifikationsdaten D3 werden von der Haupt-Schnittstelle S4 der Datenverarbeitungsanlage 4 empfangen. Das Haupt-Computerprogramm C4 der Datenverarbeitungsanlage 4 liest die Identifikationsdaten D3 ein.

Gemäss dem dritten einundzwanzigsten Alternativschritt XXIc begibt sich der Athlet A zur Messplattform 1.

Im zweiundzwanzigsten Unterschritt XXII befindet sich der Athlet A bei der Messplattform 1. Dabei weist der zweiundzwanzigste Unterschritt XXII zwei Alternativen auf, mit einem ersten zweiundzwanzigsten Alternativschritt XXIIa oder mit einem zweiten zweiundzwanzigsten Alternativschritt XXIIb.

Gemäss dem ersten zweiundzwanzigsten Alternativschritt XXIIa ermittelt das Haupt-Computerprogramm C4 der Datenverarbeitungsanlage 4, ob Identifikationsdaten D3 eingelesen worden sind und ob aktuell Messdaten D1 von der Haupt-Schnittstelle S4 der Datenverarbeitungsanlage 4 empfangen werden, also ob die Messplattform 1 frei ist. Falls Identifikationsdaten D3 eingelesen worden sind und falls aktuell keine Messdaten D1 von der Haupt-Schnittstelle S4 empfangen werden, erzeugt das Haupt-Computerprogramm C4 Übungszeitdaten D7 für eine zugeteilte Übungszeit. Die Identifikationsdaten D3 weisen eine Identifikationsnummer der ersten Rechnereinrichtung 5 des Athleten A auf. Die Datenverarbeitungsanlage 4 sendet die zugeteilte Übungszeit als Übungszeitdaten D7 über die Haupt-Schnittstelle S4 an die über die Identifikationsnummer identifizierte erste Rechnereinrichtung 5, welche die Übungszeitdaten D7 über die erste Schnittstelle S5 empfängt. Die Übungszeitdaten D7 werden dem Athleten A auf dem ersten Ausgabemittel AU5 der ersten Rechnereinrichtung 5 als zugeteilte Übungszeit ausgegeben. Der Athlet A nimmt von der zugeteilten Übungszeit Kenntnis und steigt er zur zugeteilten Übungszeit auf die Messplattform 1. Der Athlet A erhält mit der zugeteilten Übungszeit den Vorteil, dass er nicht mehr warten muss, ob die Messplattform 1 frei ist, was für den Athleten A stressfrei ist.

Gemäss dem zweiten zweiundzwanzigsten Alternativschritt XXIIb steigt der Athlet A bei freier Messplattform 1 auf die Messplattform 1. Der Athlet A quittiert seine Bereitschaft, mit den Sprungübungen zu beginnen, mit dem Startsignal. Er gibt das Startsignal über das erste Eingabemittel EI5 in Form einer Taste in die erste Rechnereinrichtung 5 ein, welches von dort über die erste Schnittstelle S5 als erste Startdaten D8 an die Datenverarbeitungsanlage 4 gesendet werden, wo die ersten Startdaten D8 über die Haupt-Schnittstelle S4 empfangen werden. Die ersten Startdaten D8 können auch eine Identifikationsnummer der ersten Rechnereinrichtung 5 zur eindeutigen Identifizierung des Athleten A umfassen. Das Haupt-Computerprogramm C4 der Datenverarbeitungsanlage 4 liest die ersten Startdaten D8 ein.

Im dreiundzwanzigsten Unterschritt XXIII führt der Athlet A Sprungübungen auf der Messplattform 1 aus. Er führt die Sprungübungen unter Anleitung der Übungsinformation U2 bis U11 aus. Dazu werden die die Übungsdaten D5 auf dem ersten Ausgabemittel AU5 als Übungsinformation U2 bis U11 ausgegeben. Beispielsweise wird zuerst die zweite Übungsinformation U2 ausgegeben und vom Athleten A als Sprungübungen ausgeführt. Daraufhin wird die dritte Übungsinformation U3 ausgegeben und vom Athleten A als Sprungübungen ausgeführt. Nun wird die vierte Übungsinformation U4 ausgegeben und vom Athleten A als Sprungübungen ausgeführt. Und die fünfte Übungsinformation U5 wird ausgegeben und vom Athleten A als Sprungübungen ausgeführt. Dann wird die sechste Übungsinformation U6 ausgegeben und vom Athleten A als Sprungübungen ausgeführt. Auch die siebte Übungsinformation U7 wird ausgegeben und vom Athleten A als Sprungübungen ausgeführt. Schliesslich wird die achte Übungsinformation U8 ausgegeben und vom Athleten A als Sprungübungen ausgeführt. Für die gemessene Sprungkraft erzeugt der Messplattform-Prozessor P1 Messdaten D1, welche über die Messplattform-Schnittstelle S1 als Messdaten D1 an die Datenverarbeitungsanlage 4 gesendet werden, wo die Messdaten D1 über die Haupt-Schnittstelle S4 empfangen werden. Die Messdaten D1 werden im Haupt-Speichermedium M4 der Datenverarbeitungsanlage 4 gespeichert.

Der dreiundzwanzigste Unterschritt XXIII weist sechs Alternativen auf, mit einem ersten dreiundzwanzigsten Alternativschritt XXIIIa oder mit einem zweiten dreiundzwanzigsten Alternativschritt XXIIIb oder mit einem dritten dreiundzwanzigsten Alternativschritt XXIIIc oder mit einem vierten dreiundzwanzigsten Alternativschritt XXIIId oder mit einem fünften dreiundzwanzigsten Alternativschritt XXIIIe oder mit einem sechsten dreiundzwanzigsten Alternativschritt XXIIIf.

Der Athlet A kann die Sprungübungen mit oder ohne Erfassung von Bildern des Athleten A ausführen. Im ersten dreiundzwanzigsten Alternativschritt XXIIIa, im zweiten dreiundzwanzigsten Alternativschritt XXIIIb und im dritten dreiundzwanzigsten Alternativschritt XXIIIc werden vom Athleten A während der Sprungübungen keine Bilder erfasst. Im vierten dreiundzwanzigsten Alternativschritt XXIIId, im fünften dreiundzwanzigsten Alternativschritt XXIIIe und im sechsten dreiundzwanzigsten Alternativschritt XXIIIf werden vom Athleten A während der Sprungübungen Bilder erfasst. Die Kamera 2 kann automatisch mit der Erfassung von Bildern des Athleten A mit dem Empfang von Startdaten D8, D8', D8'' starten und mit der Erfassung von Bildern des Athleten A mit dem Empfang von Stoppdaten D9, D9', D9'' stoppen.
- So kann die erste Rechnereinheit 5 über die erste Schnittstelle S5 erste Startdaten D8 an die Kamera-Schnittstelle S2 der Kamera 2 senden, sobald der Athlet A seine Bereitschaft, mit den Sprungübungen zu beginnen, mit dem Startsignal quittiert hat. Und die erste Rechnereinheit 5 kann über die erste Schnittstelle S5 erste Stoppdaten D9 an die Kamera-Schnittstelle S2 der Kamera 2 senden, sobald der Athlet A das Ende der Sprungübungen mit einem Stoppsignal quittiert hat.
- Auch kann die Messplattform 1 über die Messplattform-Schnittstelle S1 zweite Startdaten D8' an die Kamera-Schnittstelle S2 der Kamera 2 senden, sobald Messdaten D1 erzeugt werden. Und die Messplattform 1 kann über die Messplattform-Schnittstelle S1 zweite Stoppdaten D9' an die Kamera-Schnittstelle S2 der Kamera 2 senden, sobald keine Messdaten D1 mehr erzeugt werden.
- Oder auch die Datenverarbeitungsanlage 4 kann über die Haupt-Schnittstelle S4 dritte Startdaten D8'' an die Kamera-Schnittstelle S2 der Kamera 2 senden, sobald der Athlet A seine Bereitschaft, mit den Sprungübungen zu beginnen, mit dem Startsignal quittiert hat. Und die Datenverarbeitungsanlage 4 kann über die Haupt-Schnittstelle S4 dritte Stoppdaten D9'' an die Kamera-Schnittstelle S2 der Kamera 2 senden, sobald der Athlet A das Ende der Sprungübungen mit einem Stoppsignal quittiert hat.
Die erfassten Bilder werden über die Kamera-Schnittstelle S2 als Bilddaten D2 an die Datenverarbeitungsanlage 4 gesendet werden, wo die Bilddaten D2 über die Haupt-Schnittstelle S4 empfangen werden. Die Bilddaten D2 werden im Haupt-Speichermedium M4 der Datenverarbeitungsanlage 4 gespeichert.

Im ersten dreiundzwanzigsten Alternativschritt XXIIIa führt der Athlet A die Sprungübungen auf der Messplattform 1 nach dem Versenden von Identifikationsdaten D3 aus. Messdaten D1 der Messplattform 1, welche die Datenverarbeitungsanlage 4 zeitlich direkt nach den Identifikationsdaten D3 empfängt, werden vom Haupt-Computerprogramm C4 dem über die Identifikationsdaten D3 eindeutig identifizierten Athleten A zugeordnet. Sobald die Datenverarbeitungsanlage 4 für eine vordefinierte Zeitdauer keine Messdaten D1 mehr von der Messplattform 1 empfängt, beendet das Haupt-Computerprogramm C4 automatisch die Zuordnung der Messdaten D1 zu dem über die Identifikationsdaten D3 eindeutig identifizierten Athleten A.

Im zweiten dreiundzwanzigsten Alternativschritt XXIIIb führt der Athlet A die Sprungübungen auf der Messplattform 1 zur mit den Übungszeitdaten D7 zugeteilten Übungszeit aus. Messdaten D1 der Messplattform 1, welche die Datenverarbeitungsanlage 4 zeitlich direkt nach der mit den Übungszeitdaten D7 zugeteilten Übungszeit empfängt, werden vom Haupt-Computerprogramm C4 dem über die Identifikationsdaten D3 eindeutig identifizierten Athleten A zugeordnet. Sobald die Datenverarbeitungsanlage 4 für eine vordefinierte Zeitdauer keine Messdaten D1 mehr von der Messplattform 1 empfängt, beendet das Haupt-Computerprogramm C4 automatisch die Zuordnung der Messdaten D1 zu dem über die Identifikationsdaten D3 eindeutig identifizierten Athleten A.

Im dritten dreiundzwanzigsten Alternativschritt XXIIIc führt der Athlet A die Sprungübungen auf der Messplattform 1 nach dem Versenden von ersten Startdaten D8 aus. Messdaten D1 der Messplattform 1, welche die Datenverarbeitungsanlage 4 zeitlich direkt nach den ersten Startdaten D8 empfängt, werden vom Haupt-Computerprogramm C4 dem über die ersten Startdaten D8 eindeutig identifizierten Athleten A zugeordnet. Der Athlet A quittiert ein Ende der Sprungübungen mit einem Stoppsignal. Er gibt das Stoppsignal über das erste Eingabemittel EI5 in Form einer Taste in die erste Rechnereinrichtung 5 ein, welches von dort über die erste Schnittstelle S5 als erste Stoppdaten D9 an die Datenverarbeitungsanlage 4 gesendet werden, wo die ersten Stoppdaten D9 über die Haupt-Schnittstelle S4 empfangen werden. Die ersten Stoppdaten D9 können Athletendaten D6 zur eindeutigen Identifizierung des Athleten A umfassen. Die ersten Stoppdaten D9 können eine Identifikationsnummer der ersten Rechnereinrichtung 5 zur eindeutigen Identifizierung des Athleten A umfassen. Das Haupt-Computerprogramm C4 liest die ersten Stoppdaten D9 ein. Messdaten D1 der Messplattform 1, welche von der Datenverarbeitungsanlage 4 zeitlich nach den erste Stoppdaten D9 empfangen werden, werden vom Haupt-Computerprogramm C4 nicht mehr dem Athleten A zugeordnet. Die Quittierung des Endes der Sprungübungen mit einem Stoppsignal ist optional. Es ist auch möglich, dass sobald die Datenverarbeitungsanlage 4 für eine vordefinierte Zeitdauer keine Messdaten D1 mehr von der Messplattform 1 empfängt, dass dann das Computerprogramm C4 automatisch die Zuordnung der Messdaten D1 zu Athleten A beendet.

Im vierten dreiundzwanzigsten Alternativschritt XXIIId führt der Athlet A die Sprungübungen auf der Messplattform 1 nach dem Versenden von Identifikationsdaten D3 aus. Messdaten D1 der Messplattform 1 und Bilddaten D2 der Kamera 2, welche die Datenverarbeitungsanlage 4 zeitlich direkt nach den Identifikationsdaten D3 empfängt, werden vom Haupt-Computerprogramm C4 dem über die Identifikationsdaten D3 eindeutig identifizierten Athleten A zugeordnet. Sobald die Datenverarbeitungsanlage 4 für eine vordefinierte Zeitdauer keine Messdaten D1 mehr von der Messplattform 1 und keine Bilddaten 2 mehr von der Kamera 2 empfängt, beendet das Haupt-Computerprogramm C4 automatisch die Zuordnung der Messdaten D1 und der Bilddaten D2 zu dem über die Identifikationsdaten D3 eindeutig identifizierten Athleten A.

Im fünften dreiundzwanzigsten Alternativschritt XXIIIe führt der Athlet A die Sprungübungen auf der Messplattform 1 zur mit den Übungszeitdaten D7 zugeteilten Übungszeit aus. Messdaten D1 der Messplattform 1 und Bilddaten D2 der Kamera 2, welche die Datenverarbeitungsanlage 4 zeitlich direkt nach der mit den Übungszeitdaten D7 zugeteilten Übungszeit empfängt, werden vom Haupt-Computerprogramm C4 dem über die Identifikationsdaten D3 eindeutig identifizierten Athleten A zugeordnet. Sobald die Datenverarbeitungsanlage 4 für eine vordefinierte Zeitdauer keine Messdaten D1 mehr von der Messplattform 1 und keine Bilddaten D2 mehr von der Kamera 2 empfängt, beendet das Haupt-Computerprogramm C4 automatisch die Zuordnung der Messdaten D1 zu dem über die Identifikationsdaten D3 eindeutig identifizierten Athleten A.

Im sechsten dreiundzwanzigsten Alternativschritt XXIIIf führt der Athlet A die Sprungübungen auf der Messplattform 1 nach dem Versenden von ersten Startdaten D8 aus. Messdaten D1 der Messplattform 1 und Bilddaten D2 der Kamera 2, welche die Datenverarbeitungsanlage 4 zeitlich direkt nach den ersten Startdaten D8 empfängt, werden vom Haupt-Computerprogramm C4 dem über die ersten Startdaten D8 eindeutig identifizierten Athleten A zugeordnet. Der Athlet A quittiert ein Ende der Sprungübungen mit einem Stoppsignal. Er gibt das Stoppsignal über das erste Eingabemittel EI5 in Form einer Taste in die erste Rechnereinrichtung 5 ein, welches von dort über die erste Schnittstelle S5 als erste Stoppdaten D9 an die Datenverarbeitungsanlage 4 gesendet werden, wo die ersten Stoppdaten D9 über die Haupt-Schnittstelle S4 empfangen werden. Die ersten Stoppdaten D9 können Athletendaten D6 zur eindeutigen Identifizierung des Athleten A umfassen. Die ersten Stoppdaten D9 können eine Identifikationsnummer der ersten Rechnereinrichtung 5 zur eindeutigen Identifizierung des Athleten A umfassen. Das Haupt-Computerprogramm C4 liest die ersten Stoppdaten D9 ein. Messdaten D1 der Messplattform 1 und Bilddaten D2 der Kamera 2, welche von der Datenverarbeitungsanlage 4 zeitlich nach den erste Stoppdaten D9 empfangen werden, werden vom Haupt-Computerprogramm C4 nicht mehr dem Athleten A zugeordnet. Auch hier ist die Quittierung des Endes der Sprungübungen mit einem Stoppsignal optional. Es ist auch möglich, dass sobald die Datenverarbeitungsanlage 4 für eine vordefinierte Zeitdauer keine Messdaten D1 mehr von der Messplattform 1 empfängt, dass dann das Computerprogramm C4 automatisch die Zuordnung der Messdaten D1 zu Athleten A beendet.

Bei Kenntnis der vorliegenden Erfindung kann der Fachmann Variationen des zweiten Schrittes II realisieren. So kann die erste Rechnereinrichtung eine Smartwatch sein, so dass dem Athleten die Übungszeitdaten und die Anleitungsdaten auf einem ersten Ausgabemittel der Smartwatch ausgegeben werden und dass der Athlet das Startsignal und das Stoppsignal über ein erstes Eingabemittel der Smartwatch eingibt.

### DRITTER SCHRITT III

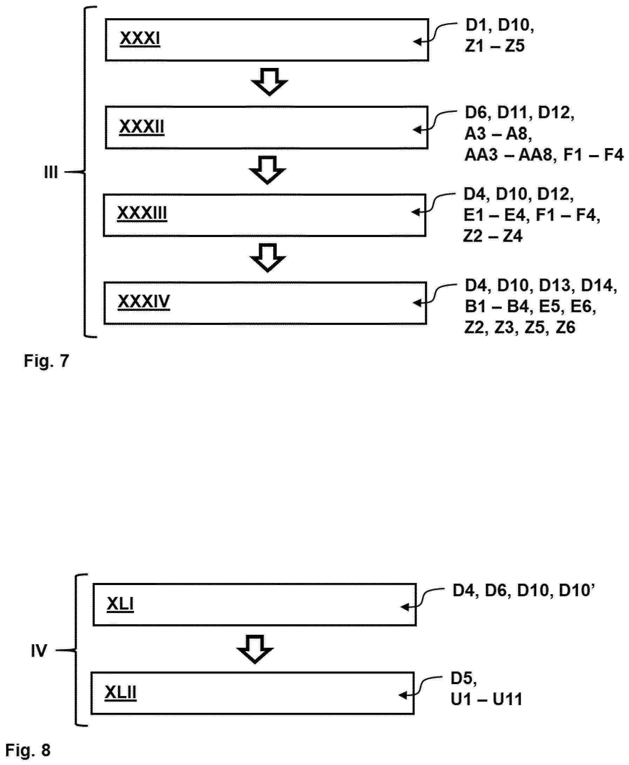

Im dritten Schritt III erfolgt eine Auswertung von Messdaten D1. Im Beispiel gemäss Fig. 2 erfolgt die Auswertung der Messdaten D1 durch das Haupt-Computerprogramm C4 der Datenverarbeitungsanlage 4. Fig. 7 zeigt den dritten Schritt III im Detail mit einem einunddreissigsten Unterschritt XXXI, mit einem zweiunddreissigsten Unterschritt XXXII, mit einem dreiunddreissigsten Unterschritt XXXIII und mit einem vierunddreissigsten Unterschritt XXXIV.

Im einunddreissigsten Unterschritt XXXI wertet das Haupt-Computerprogramm C4 die Messdaten D1 zu Leistungsdaten D10 aus. Eine Auswertung von Messdaten zu Leistungsdaten ist in der Schrift DE10040623A1 offenbart. Dabei wird mindestens eine der folgenden Ermittlungen von Leistungsdaten D10 ausgeführt:
- Das Haupt-Computerprogramm C4 dividiert die Messdaten D1 durch das Gewicht des Athleten A und ermittelt so eine Beschleunigung. Vorteilhafterweise unterteilt das Haupt-Computerprogramm C4 die Messdaten D1 zeitlich in vertikale Einzelsprünge oder vertikale Mehrfachsprünge. Für jeden der vertikalen Einzelsprünge oder vertikalen Mehrfachsprünge ermittelt das Haupt-Computerprogramm C4 eine Beschleunigung. Für mehrere vertikale Einzelsprünge oder vertikalen Mehrfachsprünge ermittelt das Haupt-Computerprogramm C4 einen Mittelwert der Beschleunigung. Der Mittelwert der Beschleunigungen wird Sprungbeschleunigungsinformation Z1 genannt.
- Das Haupt-Computerprogramm C4 integriert die Messdaten D1 einmal über die Zeit und ermittelt so eine Geschwindigkeit. Vorteilhafterweise unterteilt das Haupt-Computerprogramm C4 die Messdaten D1 zeitlich in vertikale Einzelsprünge oder vertikale Mehrfachsprünge. Für jeden der vertikalen Einzelsprünge oder vertikalen Mehrfachsprünge ermittelt das Haupt-Computerprogramm C4 eine Geschwindigkeit. Für mehrere vertikale Einzelsprünge oder vertikale Mehrfachsprünge ermittelt das Haupt-Computerprogramm C4 einen Mittelwert der Geschwindigkeit. Der Mittelwert der Geschwindigkeit wird Sprunggeschwindigkeitsinformation Z2 genannt.
- Das Haupt-Computerprogramm C4 ermittelt aus den Messdaten D1 von mehreren vertikalen Einzelsprüngen oder vertikalen Mehrfachsprüngen einen Mittelwert einer Sprungkraft. Vorteilhafterweise unterteilt das Haupt-Computerprogramm C4 die Messdaten D1 zeitlich in vertikale Einzelsprünge oder vertikale Mehrfachsprünge. Für jeden der vertikalen Einzelsprünge oder vertikalen Mehrfachsprünge ermittelt das Haupt-Computerprogramm C4 eine Sprungkraft. Für mehrere vertikale Einzelsprünge oder vertikale Mehrfachsprünge mittelt das Haupt-Computerprogramm C4 die Sprungkraft zu einem Mittelwert der Sprungkraft. Der Mittelwert der Sprungkraft wird Sprungkraftinformation Z3 genannt.
- Das Haupt-Computerprogramm C4 multipliziert den Mittelwert der Sprungkraft mit dem Mittelwert der Geschwindigkeit und ermittelt so einen Mittelwert der Leistung. Der Mittelwert der Leistung wird Sprungleistungsinformation Z4 genannt.
- Das Haupt-Computerprogramm C4 integriert die Messdaten D1 zweimal über die Zeit und ermittelt so eine Sprunghöhe. Vorteilhafterweise unterteilt das Haupt-Computerprogramm C4 die Messdaten D1 zeitlich in vertikale Einzelsprünge oder vertikale Mehrfachsprünge. Für jeden der vertikalen Einzelsprünge oder vertikalen Mehrfachsprünge ermittelt das Haupt-Computerprogramm C4 eine Sprunghöhe. Für mehrere vertikale Einzelsprünge oder vertikale Mehrfachsprünge ermittelt das Haupt-Computerprogramm C4 einen Mittelwert der Sprunghöhe. Der Mittelwert der Sprunghöhe wird Sprunghöheninformation Z5 genannt.

Die vom Haupt-Computerprogramm C4 ermittelten Leistungsdaten D10 werden im Haupt-Speichermedium M4 gespeichert.

Im Haupt-Speichermedium M4 sind eine grosse Anzahl von empirisch ermittelten anderen Athletendaten D11 gespeichert. Beispielsweise sind mehr als eine Million andere Athletendaten D11 gespeichert. Die anderen Athletendaten D11 umfassen mindestens eine der folgenden Informationen:
- Eine dritte andere Athleteninformation AA3 ist ein Geschlecht von anderen Athleten.
- Eine vierte andere Athleteninformation AA4 ist ein Alter von anderen Athleten.
- Eine fünfte andere Athleteninformation AA5 ist eine Grösse von anderen Athleten.
- Eine sechste andere Athleteninformation AA6 ist ein Gewicht von anderen Athleten.
- Eine siebte andere Athleteninformation AA7 ist eine Sportart von anderen Athleten.
- Eine achte andere Athleteninformation AA8 ist eine frei bestimmbare Sporteigenschaft von anderen Athleten wie "Stürmer", "Linksfüsser", "Junior", usw.

Im zweiunddreissigsten Unterschritt XXXII filtert das Haupt-Computerprogramm C4 die anderen Athletendaten D11 mit den Athletendaten D6. Die Athletendaten D6 umfassen mindestens eine der folgenden Informationen:
- Eine dritte Athleteninformation A3 ist ein Geschlecht des Athleten A.
- Eine vierte Athleteninformation A4 ist ein Alter des Athleten A.
- Eine fünfte Athleteninformation A5 ist eine Grösse des Athleten A.
- Eine sechste Athleteninformation A6 ist ein Gewicht des Athleten A.
- Eine siebte Athleteninformation A7 ist eine Sportart des Athleten A.
- Eine achte Athleteninformation A8 ist eine frei bestimmbare Sporteigenschaft des Athleten A.

Das Haupt-Computerprogramm C4 ermittelt aus den anderen Athletendaten D11 vergleichbare andere Athletendaten D12. Vergleichbare andere Athletendaten D12 erfüllen mindestens eines der folgenden Kriterien:
- Dass die dritte andere Athleteninformation AA3 der dritten Athleteninformation A3 entspricht. Das Geschlecht der dritten anderen Athleteninformation AA3 muss dem Geschlecht der dritten Athleteninformation A3 entsprechen.
- Dass die vierte andere Athleteninformation AA4 der vierten Athleteninformation A4 entspricht. Das Alter der vierten anderen Athleteninformation AA4 entspricht mit einer Altersspanne von beispielsweise +/-2Jahren dem Alter der vierten Athleteninformation A4.
- Dass die fünfte andere Athleteninformation AA5 der fünften Athleteninformation A5 entspricht. Die Grösse der fünften anderen Athleteninformation AA5 entspricht mit einer Grössenspanne von beispielsweise +/-5cm der Grösse der fünften Athleteninformation A5.
- Dass die sechste andere Athleteninformation AA6 der sechsten Athleteninformation A6 entspricht. Das Gewicht der sechsten anderen Athleteninformation AA6 entspricht mit einer Gewichtsspanne von beispielsweise +/-2kg dem Gewicht der sechsten Athleteninformation A6.
- Dass die siebte andere Athleteninformation AA7 der siebten Athleteninformation A7 entspricht. Die Sportart der siebten anderen Athleteninformation AA7 muss der Sportart der siebten Athleteninformation A7 entsprechen.
- Dass die achte andere Athleteninformation AA8 der achten Athleteninformation A8 entspricht. Die frei bestimmbare Sporteigenschaft der achten anderen Athleteninformation AA8 muss der frei bestimmbaren Sporteigenschaft der achten Athleteninformation A8 entsprechen.
Im Ergebnis der Filterung verbleiben von der mehr als eine Million anderen Athletendaten D11 beispielsweise mehrere hundert vergleichbare andere Athletendaten D12.

Die vergleichbaren andere Athletendaten D12 umfassen mindestens eine der folgenden vergleichbaren anderen Athleteninformationen F1 - F4:
- Eine Sprungkraftinformation F1 der anderen Athleten.
- Eine Sprunggeschwindigkeitsinformation F2 der anderen Athleten.
- Eine Sprungleistungsinformation F3 der anderen Athleten.
- Eine Sprungkraftinformation F1 über eine Differenz der Sprungkraft der beiden unteren Extremitäten der anderen Athleten. Es ist nicht ungewöhnlich, dass die beiden unteren Extremitäten eines Athleten eine Differenz von beispielsweise 4% in der Sprungkraft aufweisen.

Im dreiunddreissigsten Unterschritt XXXIII ermittelt das Haupt-Computerprogramm C4 für die Leistungsdaten D10 Expertendaten D4. Dazu vergleicht das Haupt-Computerprogramm C4 Leistungsdaten D10 des Athleten A mit den vergleichbaren anderen Athletendaten D12. Das Ergebnis des Vergleiches sind die folgenden Expertendaten D4:
- Leistungsdaten D10 von der Sprungkraftinformation Z3 des Athleten A werden mit den vergleichbaren anderen Athletendaten D12 von einer Sprungkraftinformation F1 der anderen Athleten verglichen, und im Vergleichsergebnis werden Expertendaten D4 von einer ersten Experteninformation E1 über eine potenziell möglichen Sprungkraft des Athleten A ermittelt. Die erste Experteninformation E1 besagt, wie nahe die Sprungkraftinformation Z3 des Athleten A der Sprungkraftinformation F1 der anderen Athleten kommt.
- Leistungsdaten D10 von der Sprunggeschwindigkeitsinformation Z2 des Athleten A werden mit vergleichbaren anderen Athletendaten D12 von einer Sprunggeschwindigkeitsinformation F2 der anderen Athleten verglichen, und im Vergleichsergebnis werden Expertendaten D4 von einer zweiten Experteninformation E2 über eine potenziell mögliche Sprunggeschwindigkeit des Athleten A ermittelt. Die zweite Experteninformation E2 besagt, wie nahe die Sprunggeschwindigkeitsinformation Z2 des Athleten A der Sprunggeschwindigkeitsinformation F2 der anderen Athleten kommt.
- Leistungsdaten D10 von der Sprungleistungsinformation Z4 des Athleten A werden mit vergleichbaren anderen Athletendaten D12 von einer Sprungleistungsinformation F3 der anderen Athleten verglichen, im Vergleichsergebnis werden Expertendaten D4 von einer dritten Experteninformation E3 über eine potenziell möglichen Sprungleistung des Athleten A ermittelt. Die dritte Experteninformation E3 besagt, wie nahe die Sprungleistungsinformation Z4 des Athleten A der Sprungleistungsinformation F3 der anderen Athleten kommt.
- Leistungsdaten D1 von der Sprungkraftinformation Z3 des Athleten A werden mit vergleichbaren anderen Athletendaten D12 von einer Sprungkraftinformation F4 über eine Differenz der Sprungkraft der beiden unteren Extremitäten der anderen Athleten verglichen, und im Vergleichsergebnis werden Expertendaten D4 von einer vierten Experteninformation E4 über eine Differenz der Sprungkraft der beiden unteren Extremitäten des Athleten A ermittelt. Die vierte Experteninformation E4 besagt, wie stark die Differenz der Sprungkraft der beiden unteren Extremitäten des Athleten A von der Differenz der Sprungkraft der beiden unteren Extremitäten der anderen Athleten abweicht.

Im vierunddreissigsten Unterschritt XXXIV ermittelt das Haupt-Computerprogramm C4 für die Leistungsdaten D10 weitere Expertendaten D4. Dazu sind im Haupt-Speichermedium M4 biometrische Daten D13 eines Athleten und medizinische Daten D14 über ein zukünftiges Verletzungsrisiko eines Athleten gespeichert.

Die biometrischen Daten D13 sind das Ergebnis von biometrischen Modellberechnungen. Die biometrischen Daten D13 umfassen mindestens eine der folgenden biometrischen Informationen B1 - B3:
- Eine maximal möglichen Sprungkraftinformation B1 eines Athleten.
- Eine maximal mögliche Sprunggeschwindigkeitsinformation B2 eines Athleten.
- Eine maximal mögliche Sprungleistungsinformation B3 eines Athleten.

Die medizinischen Daten D14 über ein zukünftiges Verletzungsrisiko eines Athleten berücksichtigen eine Differenz der Sprungkraft der beiden unteren Extremitäten eines Athleten. Denn eine zu grosse Differenz in der Sprungkraft der beiden unteren Extremitäten eines Athleten von beispielsweise mehr als 8% beinhaltet ein zukünftiges Verletzungsrisiko für den Athleten wie ein Bänderriss, ein Muskelriss, usw.

Das Haupt-Computerprogramm C4 vergleicht Leistungsdaten D10 des Athleten A mit den biometrischen Daten D13 und den medizinischen Daten D14. Das Ergebnis des Vergleiches sind die folgenden Expertendaten D4:
- Leistungsdaten D10 von einer Sprungkraftinformation Z3 des Athleten A werden mit Leistungsdaten D10 von einer Sprunggeschwindigkeitsinformation Z2 des Athleten A in Beziehung gesetzt und Leistungsdaten 10 von einer Sprungkraft-Sprunggeschwindigkeitsinformation Z6 des Athleten A gebildet. Biometrische Daten D13 von einer maximal möglichen Sprungkraftinformation B1 eines Athleten werden mit biometrischen Daten D13 von einer maximal möglichen Sprunggeschwindigkeitsinformation B2 eines Athleten in Beziehung gesetzt und biometrische Daten 13 von einer Sprungkraft-Sprunggeschwindigkeitsinformation B4 eines Athleten gebildet. Die Leistungsdaten 10 von der Sprungkraft-Sprunggeschwindigkeitsinformation Z6 des Athleten A werden nun mit den biometrischen Daten D13 von der Sprungkraft-Sprunggeschwindigkeitsinformation B4 eines Athleten verglichen, im Vergleichsergebnis werden Expertendaten D4 von einer fünften Experteninformation E5 über eine Sprungkraft-Sprunggeschwindigkeit-Beziehung des Athleten A ermittelt. Die fünfte Experteninformation E5 besagt, wie ausgeglichen die Sprungkraftinformation Z3 und die Sprunggeschwindigkeitsinformation Z2 des Athleten A entwickelt sind.
- Die Expertendaten D4 der vierten Experteninformation E4 über eine Differenz der Sprungkraft der beiden unteren Extremitäten des Athleten A werden mit den medizinischen Daten D14 verglichen und im Vergleichsergebnis werden Expertendaten D4 von einer sechsten Experteninformation E6 über eine zukünftige Verletzungsrisiko des Athleten A ermittelt.

Die ermittelten Leistungsdaten D10 und die ermittelten Expertendaten D4 werden im Haupt-Speichermedium M4 der Datenverarbeitungsanlage 4 gespeichert.

Leistungsdaten, welche die Datenverarbeitungsanlage 4 in der Vergangenheit über die Haupt-Schnittstelle S4 empfangen hat, und welche im Haupt-Speichermedium M4 als historische Leistungsdaten D10' gespeichert sind, weisen historische Leistungsinformation L1 bis L6 auf. Die historische Leistungsinformation L1 bis L6 umfasst eine Leistungsinformation zu historischen Sprungübungen wie eine historisch ermittelte Sprungbeschleunigungsinformation, eine historisch ermittelte Sprunggeschwindigkeitsinformation, eine historisch ermittelte Sprungkraftinformation, eine historisch ermittelte Sprungleistungsinformation und eine historisch ermittelte Sprunghöheinformation. Im Sinne der Erfindung bedeutet das Adjektiv "historisch", dass die damit beschriebene Leistungsinformation sich auf die Vergangenheit bezieht. Die Leistungsinformation L1 bis L6 war also in der Vergangenheit aktuell.

Die historische Leistungsinformation L1 bis L6 ist eine alphanumerische Zeichenfolge, ein Zahlenwert, ein Graph, usw. Beispiele für die historische Leistungsinformation L1 bis L6 sind:
- Eine erste historische Leistungsinformation L1 umfasst Angaben zu den letzten ausgeführten Sprungübungen (beispielsweise vor einer Woche).
- Eine historische zweite Leistungsinformation L2 umfasst Angaben zu den vorletzten ausgeführten Sprungübungen (beispielsweise vor zwei Wochen).
- Eine historische dritte Leistungsinformation L3 umfasst Angaben zu den vorvorletzten ausgeführten Sprungübungen (beispielsweise vor drei Wochen).
- Eine historische vierte Leistungsinformation L4 umfasst Angaben zu den viertletzten ausgeführten Sprungübungen (beispielsweise vor vier Wochen).
- Eine historische fünfte Leistungsinformation L5 umfasst Angaben zu den fünftletzten ausgeführten Sprungübungen (beispielsweise vor fünf Wochen).
- Eine historische sechste Leistungsinformation L6 umfasst Angaben zu den sechstletzten ausgeführten Sprungübungen (beispielsweise vor sechs Wochen).

### VIERTER SCHRITT IV

Im vierten Schritt IV erfolgt eine Kenntnisnahme der Leistungsdiagnostik durch den Athleten A und/oder den Betreuer B. Es sind der Athlet A an der ersten Rechnereinrichtung 5 und der Betreuer B an der zweiten Rechnereinrichtung 6, welche von der Leistungsdiagnostik Kenntnis nehmen. Fig. 8 zeigt den vierten Schritt IV im Detail mit einem einundvierzigsten Unterschritt XLI und mit einem zweiundvierzigsten Unterschritt XLII.

Im einundvierzigsten Unterschritt XLI empfängt die erste Rechnereinrichtung 5 Athletendaten D6, Leistungsdaten D10, historische Leistungsdaten D10' und Expertendaten D4 über die erste Schnittstelle S5 und die zweite Rechnereinrichtung 6 empfängt Athletendaten D6, Leistungsdaten D10, historische Leistungsdaten D10' und Expertendaten D4 über die zweite Schnittstelle S6.

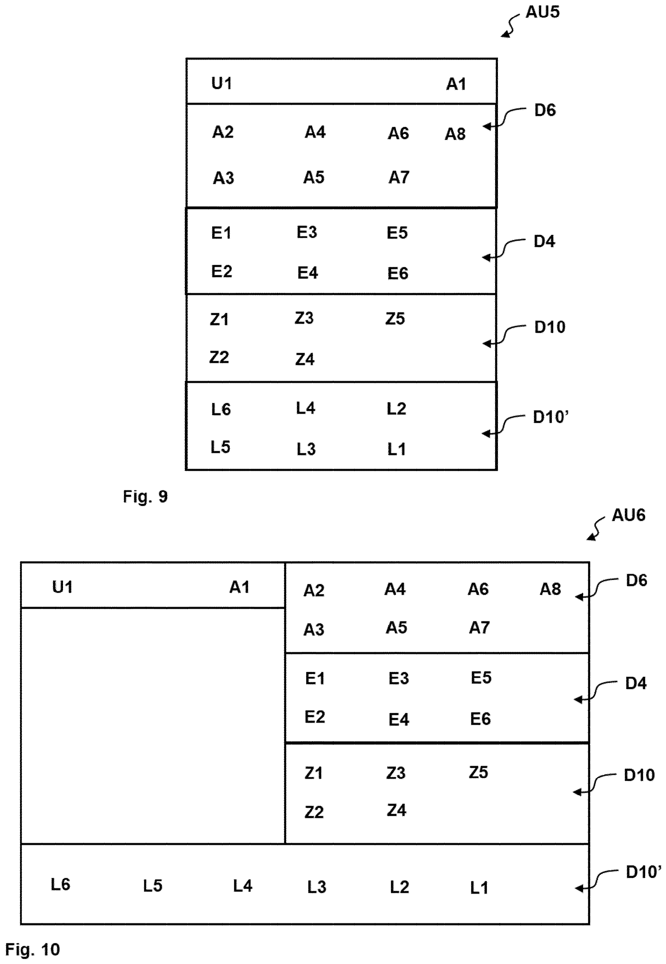

Gemäss Fig. 9 liest das erste Computerprogramm C5 die Athletendaten D6, die Leistungsdaten D10, die historischen Leistungsdaten D10' und die Expertendaten D4 ein und gibt sie auf dem ersten Ausgabemittel AU5 der ersten Rechnereinrichtung 5 aus. Die Gesamtheit der Athletendaten D6 wird als Athleteninformation A1 bis A8 ausgegeben. Leistungsdaten D10 werden als Leistungsinformation Z1 bis Z5 ausgegeben. Die Gesamtheit der historischen Leistungsdaten D10' wird als historische Leistungsinformation L1 bis L6 ausgegeben. Und die Gesamtheit der Expertendaten D4 wird als Experteninformation E1 bis E6 ausgegeben.

Gemäss Fig. 10 liest das zweite Computerprogramm C6 die Athletendaten D6, die Leistungsdaten D10, die historischen Leistungsdaten D10' und die Expertendaten D4 ein und gibt sie auf dem zweiten Ausgabemittel AU6 der zweiten Rechnereinrichtung 6 aus. Die Gesamtheit der Athletendaten D6 wird als Athleteninformation A1 bis A8 ausgegeben. Die Leistungsdaten D10 werden als Leistungsinformation Z1 bis Z5 ausgegeben. Die Gesamtheit der historischen Leistungsdaten D10' wird als historische Leistungsinformation L1 bis L6 ausgegeben. Und die Gesamtheit der Expertendaten D4 wird als Experteninformation E1 bis E6 ausgegeben.

Dem Athleten A und/oder dem Betreuer B wird/werden somit die Leistungsinformation Z1 bis Z5 mit einer Experteninformation E1 bis E6 angezeigt. Mit der Experteninformation E1 bis E6 können der Athlet A und/oder der Betreuer B die Leistungsinformation Z1 bis Z5 interpretieren.

Fig. 11 ist ein Beispiel einer graphischen Darstellung der fünften Experteninformation E5 über die Sprungkraft-Sprunggeschwindigkeits-Beziehung des Athleten A auf dem Ausgabemittel AU5, AU6 der ersten oder zweiten Rechnereinrichtung 5, 6. Auf der Abszisse des Graphen ist die Sprunggeschwindigkeitsinformation Z2 des Athleten A aufgetragen, und auf der Ordinate des Graphen ist die Sprungkraftinformation Z3 des Athleten A aufgetragen. Die Sprungkraft-Sprunggeschwindigkeitsinformation Z6 des Athleten A ist als durchgezogene gerade Linie eingetragen. Die Sprungkraft-Sprunggeschwindigkeitsinformation B4 eines Athleten ist als gestrichelt gezeichnete gerade Linie eingetragen.

Der Vergleich der Sprungkraft-Sprunggeschwindigkeitsinformation Z6 des Athleten A mit der Sprungkraft-Sprunggeschwindigkeits-information B4 eines Athleten liefert im Beispiel nach Fig. 11 die fünfte Experteninformation E5 mit der Aussage, dass die Sprunggeschwindigkeitsinformation Z2 zu schwach ausgeprägt ist, während die Sprungkraftinformation Z3 zu stark ausgeprägt ist. Der Athlet A und der Betreuer B erhalten somit mit der fünften Experteninformation E5 die Empfehlung, den Geschwindigkeitsanteil an den Sprungübungen zu verstärken, und den Kraftanteil an den Sprungübungen zu reduzieren.

Im zweiundvierzigsten Unterschritt XLII kommunizieren der Betreuer B und der Athlet A über die Durchführung von Sprungübungen an einem neuen Übungsdatum U1. Das Ergebnis der Kommunikation sind neue Übungsdaten D5 mit mindestens einer Übungsinformation U1 bis U11. Die neuen Übungsdaten D5 berücksichtigen die Kenntnisnahme der Leistungsinformation Z1 bis Z5 und der Experteninformation E1 bis E6 durch den Betreuer B und den Athleten A. Die Übungsdaten D5 sind im ersten Speichermedium M5 der ersten Rechnereinrichtung 5 und im zweiten Speichermedium M6 der zweiten Rechnereinrichtung 6 gespeichert. Die neuen Übungsdaten D5 sind eine Auswahl der gespeicherten Übungsdaten D5, mit Angaben zu Sprungübungen, welche Sprungübungen vom Athleten A am neuen Übungsdatum U1 ausgeführt werden sollen.

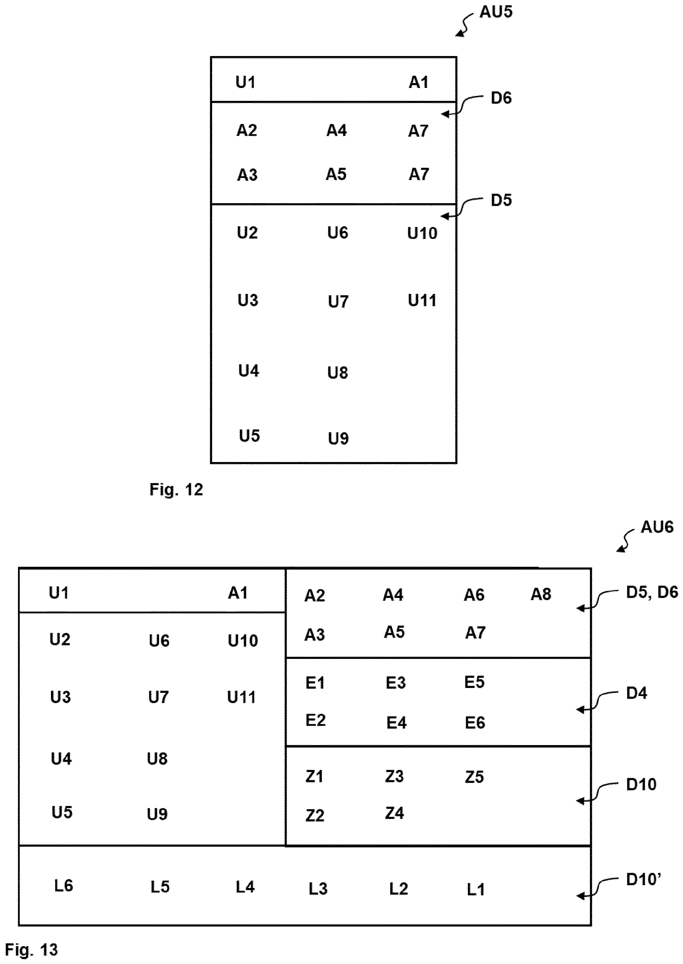

Fig. 12 zeigt eine schematische Darstellung der Athletendaten D6 und der Übungsdaten D5, welche vom ersten Computerprogramm C5 auf dem ersten Ausgabemittel AU5 der ersten Rechnereinrichtung 5 ausgegeben werden. Die Gesamtheit der Athletendaten D6 wird als Athleteninformation A1 bis A8 ausgegeben. Die Gesamtheit der Übungsdaten D5 wird als Übungsinformation U1 bis U11 ausgegeben.

Fig. 13 ist eine schematische Darstellung der Athletendaten D6, der neuen Übungsdaten D5, der Leistungsdaten D10, der Expertendaten D4 und der historischen Leistungsdaten D10', welche vom zweiten Computerprogramm C6 auf dem zweiten Ausgabemittel AU6 der zweiten Rechnereinrichtung 6 ausgegeben werden. Die Gesamtheit der Athletendaten D6 wird als Athleteninformation A1 bis A8 ausgegeben. Die Gesamtheit der neuen Übungsdaten D5 wird als Übungsinformation U1 bis U11 ausgegeben. Die Leistungsdaten D10 werden als Leistungsinformation Z1 bis Z5 ausgegeben. Die Gesamtheit der historischen Leistungsdaten D10' wird als historische Leistungsinformation L1 bis L6 ausgegeben.

Bei Kenntnis der vorliegenden Erfindung kann der Fachmann vielfältige Variationen der Beispiele realisieren. So kann die Datenverarbeitungsanlage mit der ersten Rechnereinrichtung identisch sein. In diesem Fall gibt es keine eigenständige Datenverarbeitungsanlage, sondern nur eine erste Rechnereinrichtung. Die erste Rechnereinrichtung weist dann mindestens ein erstes Speichermedium für digitale Daten auf und im ersten Prozessor ist dann das Haupt-Computerprogramm geladen und wird vom ersten Prozessor ausgeführt wird. Das ausgeführte Haupt-Computerprogramm veranlasst den ersten Prozessor dann den dritten Schritt des Verfahrens auszuführen.

### Bezugszeichenliste

- 1: Messplattform
- 2: Kamera
- 3: Datenübertragungseinrichtung
- 4: Datenverarbeitungsanlage
- 5: erste Rechnereinrichtung
- 6: zweite Rechnereinrichtung
- 10: Vorrichtung
- A: Athlet
- A1 bis A8: Athleteninformation
- AA3 bis AA8: andere Athleteninformation
- AU4 - AU6: Ausgabemittel
- B: Betreuer
- B1 - B4: biometrische Information
- BS: Bildsensor
- C4 - C6: Computerprogramm
- D1: Messdaten
- D2: Bilddaten
- D3: Identifikationsdaten
- D4: Expertendaten
- D5: Übungsdaten
- D6: Athletendaten
- D7: Übungszeitdaten
- D8, D8', D8'': Startdaten
- D9, D9', D9'': Stoppdaten
- D10, D10': Leistungsdaten
- D11: andere Athletendaten
- D12: vergleichbare andere Athletendaten
- D13: biometrische Daten
- D14: medizinische Daten
- E: Entwickler
- E1 bis E6: Experteninformation
- EI4 - EI6: Eingabemittel
- F1 - F4: vergleichbare andere Athleteninformation
- KS bis KS''': Kraftsensor
- L1 bis L6: historische Leistungsinformation
- M4 - M6: Speichermedium
- P1, P2, P4 - P6: Prozessor
- S1, S2, S4 - S6: Schnittstelle
- U1 bis U11: Übungsinformation
- I bis IV: Schritte
- XI bis XIII: erste Unterschritte
- XXI bis XXIII: zweite Unterschritte
- XXIa bis XXIc: einundzwanzigste Alternativschritte
- XXIIa, XXIIb: zweiundzwanzigste Alternativschritte
- XXIIIa bis XXIIIf: dreiundzwanzigste Alternativschritte
- XXXI bis XXXIV: dritte Unterschritte
- XLI, XLII: vierte Unterschritte
- Z1 bis Z6: Leistungsinformation

## Patentansprüche

1. Verfahren zur Kraftmessung von mindestens einem Athleten (A) unter Verwendung einer Messplattform (1);
wobei in einem ersten Schritt (I) Übungsdaten (D5) für Sprungübungen erzeugt werden, unter Verwendung einer Datenverarbeitungsanlage (4), von welcher die Übungsdaten (D5) an eine erste Rechnereinrichtung (5) gesendet werden;
wobei in einem zweiten Schritt (II) Sprungübungen angeleitet werden, unter Verwendung der ersten Rechnereinrichtung (5) durch den Athleten (A), auf welcher die Übungsdaten (D5) an den Athleten (A) ausgegeben werden; welcher Athlet (A) Sprungübungen auf der Messplattform (1) ausführt; von welcher eine Sprungkraft der Sprungübungen gemessen wird, von welcher für die gemessene Sprungkraft Messdaten (D1) erzeugt werden, und von welcher die Messdaten (D1) an die Datenverarbeitungsanlage (4) gesendet werden;
wobei in einem dritten Schritt (III) die Messdaten (D1) ausgewertet werden, unter Verwendung der Datenverarbeitungsanlage (4), auf welcher die Messdaten (D1) zu Leistungsdaten (D10) ausgewertet werden, auf welcher für die Leistungsdaten (D10) Expertendaten (D4) ermittelt werden, und von welcher die Leistungsdaten (D10) und die Expertendaten (D4) an mindestens eine der Folgenden, die erste Rechnereinrichtung (5) und eine zweite Rechnereinrichtung (6) gesendet werden; und
wobei in einem vierten Schritt (IV) die Leistungsdaten (D10) und die Expertendaten (D4) auf mindestens einer der Folgenden, der ersten Rechnereinrichtung (5) und der zweiten Rechnereinrichtung (6) ausgegeben werden.

2. Verfahren nach Anspruch 1, **dadurch gekennzeichnet, dass** im zweiten Schritt (II) die Messplattform (1) von der ersten Rechnereinrichtung (5) identifiziert wird; dass von der ersten Rechnereinrichtung (5) Identifikationsdaten (D3) mit einer Identifikationsnummer der identifizierten Messplattform (1) und mit einer Identifikationsnummer der ersten Rechnereinrichtung (5) an die Datenverarbeitungsanlage (4) gesendet werden; dass die Messplattform (1) von der Datenverarbeitungsanlage (4) über die Identifikationsnummer der Messplattform (1) eindeutig identifiziert wird; dass der Athlet (A) von der Datenverarbeitungsanlage (4) über die Identifikationsnummer der ersten Rechnereinrichtung (5) eindeutig identifiziert wird; und dass von der Datenverarbeitungsanlage (4) Messdaten (D1) der Messplattform (1), welche zeitlich direkt nach den Identifikationsdaten (D3) empfangen werden, dem Athleten (A) zuordnet.

3. Verfahren nach Anspruch 1, **dadurch gekennzeichnet, dass** im zweiten Schritt (II) von der ersten Rechnereinrichtung (5) Identifikationsdaten (D3) mit einer die Identifikationsnummer der ersten Rechnereinrichtung (5) ausgesendet werden; dass die Identifikationsdaten (D3) in einer Nähe der Messplattform (1) empfangen und an die Datenverarbeitungsanlage (4) gesendet werden; dass der Athlet (A) von der Datenverarbeitungsanlage (4) über die Identifikationsnummer der ersten Rechnereinrichtung (5) eindeutig identifiziert wird; dass von der Datenverarbeitungsanlage (4) dem Athleten (A) eine Übungszeit zugeteilt wird; dass die zugeteilte Übungszeit als Übungszeitdaten (D7) an die zweite Rechnereinrichtung (6) gesendet werden; dass dem Athleten (A) die Übungszeitdaten (D7) auf der ersten Rechnereinrichtung (5) als Übungszeit ausgegeben werden; dass der Athlet (A) zur zugeteilten Übungszeit auf die Messplattform (1) steigt; und dass und dass von der Datenverarbeitungsanlage (4) Messdaten (D1) der Messplattform (1), welche zeitlich direkt nach der zugeteilten Übungszeit empfangen werden, dem Athleten (A) zuordnet werden.

4. Verfahren nach Anspruch 1, **dadurch gekennzeichnet, dass** im zweiten Schritt (II) vom Athleten (A) ein Startsignal in die erste Rechnereinrichtung (5) eingegeben wird; dass das Stoppsignal als Startdaten (D8) an die Datenverarbeitungsanlage (4) gesendet wird; dass der Athlet (A) von der Datenverarbeitungsanlage (4) über die Startdaten (D8) eindeutig identifiziert wird; und dass Messdaten (D1), welche von der Datenverarbeitungsanlage (4) zeitlich nach den Startdaten (D8) empfangen werden, von ihr dem Athleten (A) zugeordnet werden.

5. Verfahren nach einem der Ansprüche 1 bis 4, **dadurch gekennzeichnet, dass** im zweiten Schritt (II) von einer Kamera (2) Sprungübungen des Athleten (A) als Bilder erfasst werden und für die erfassten Bilder Bilddaten (D2) erzeugt werden; und dass die Bilddaten (D2) von der Kamera (2) an die Datenverarbeitungsanlage (4) gesendet werden.

6. Verfahren nach einem der Ansprüche 1 bis 5, **dadurch gekennzeichnet, dass** im dritten Schritt (III) mindestens eine der folgenden Ermittlungen von Leistungsdaten (D10) ausgeführt wird:
- dass die Messdaten (D1) durch ein Gewicht des Athleten (A) dividiert werden und Leistungsdaten (D10) von einer Sprungbeschleunigungsinformation (Z1) ermittelt werden;
- dass die Messdaten (D1) einmal über die Zeit integriert werden und Leistungsdaten (D10) von einer Sprunggeschwindigkeitsinformation (Z2) ermittelt werden;
- dass die Messdaten (D1) gemittelt werden und Leistungsdaten (D10) von einer Sprungkraftinformation (Z3) ermittelt werden;
- dass die Messdaten (D1) einmal über die Zeit integriert werden und Leistungsdaten (D10) von einer Sprunggeschwindigkeitsinformation (Z2) ermittelt werden, dass die Messdaten (D1) gemittelt werden und Leistungsdaten (D10) von einer Sprungkraftinformation (Z3) ermittelt werden, und dass die Sprungkraftinformation (Z3) mit der Sprunggeschwindigkeitsinformation (Z2) multipliziert wird und Leistungsdaten (D10) von einer Sprungleistungsinformation (Z4) ermittelt werden; und
- dass die Messdaten (D1) zweimal über die Zeit integriert werden und Leistungsdaten (D10) von einer Sprunghöheninformation (Z5) ermittelt werden.

7. Verfahren nach Anspruch 6, **dadurch gekennzeichnet, dass** im vierten Schritt (IV) Leistungsdaten (D10) als mindestens eine der folgenden Leistungsinformationen:
- Sprungbeschleunigungsinformation (Z1),
- Sprunggeschwindigkeitsinformation (Z2),
- Sprungkraftinformation (Z3),
- Sprungleistungsinformation (Z4), und
- Sprunghöheninformation (Z5),
auf einem ersten Ausgabemittel (AU5) der ersten Rechnereinrichtung (5) und/oder auf einem zweiten Ausgabemittel (AU6) der zweiten Rechnereinrichtung (6) ausgegeben werden.

8. Verfahren nach Anspruch 7, **dadurch gekennzeichnet, dass** im dritten Schritt (III) Athletendaten (D6) bereitgestellt werden, welche Athletendaten (D6) mindestens eine der folgenden Informationen umfassen:
- eine dritte Athleteninformation (A3) ist ein Geschlecht des Athleten (A);
- eine vierte Athleteninformation (A4) ist ein Alter des Athleten (A);
- eine fünfte Athleteninformation (A5) ist eine Grösse des Athleten (A);
- eine sechste Athleteninformation (A6) ist ein Gewicht des Athleten (A);
- eine siebte Athleteninformation (A7) ist eine Sportart des Athleten (A); und
- eine achte Athleteninformation (A8) ist eine frei bestimmbare Sporteigenschaft des Athleten (A);
dass andere Athletendaten (D11) bereitgestellt werden; welchen anderen Athletendaten (D11) mindestens eine der folgenden Informationen umfassen:
- eine dritte andere Athleteninformation (AA3) ist ein Geschlecht von anderen Athleten;
- eine vierte andere Athleteninformation (AA4) ist ein Alter von anderen Athleten;
- eine fünfte andere Athleteninformation (AA5) ist eine Grösse von anderen Athleten;
- eine sechste andere Athleteninformation (AA6) ist ein Gewicht von anderen Athleten;
- eine siebte andere Athleteninformation (AA7) ist eine Sportart von anderen Athleten; und
- eine achte andere Athleteninformation (AA8) ist eine frei bestimmbare Sporteigenschaft von anderen Athleten;
und dass die anderen Athletendaten (D11) zu vergleichbaren andere Athletendaten (D12) gefiltert werden, welche vergleichbare anderen Athletendaten (D12) mindestens eines der folgenden Kriterien erfüllen:
- dass die dritte andere Athleteninformation (AA3) der dritten Athleteninformation (A3) entspricht;
- dass die vierte andere Athleteninformation (AA4) der vierten Athleteninformation (A4) entspricht;
- dass die fünfte andere Athleteninformation (AA5) der fünften Athleteninformation (A5) entspricht;
- dass die sechste andere Athleteninformation (AA6) der sechsten Athleteninformation (A6) entspricht;
- dass die siebte andere Athleteninformation (AA7) der siebten Athleteninformation (A7) entspricht; und
- dass die achte andere Athleteninformation (AA8) der achten Athleteninformation (A8) entspricht.

9. Verfahren nach Anspruch 8, **dadurch gekennzeichnet, dass** die im dritten Schritt (III) ermittelten vergleichbaren anderen Athletendaten (D12) eine Sprungkraftinformation (F1) der anderen Athleten umfassen und die ebenfalls im dritten Schritt (III) ermittelten Leistungsdaten (D10) von einer Sprungkraftinformation (Z3) mit den vergleichbaren anderen Athletendaten (D12) von einer Sprungkraftinformation (F1) der anderen Athleten verglichen werden und Expertendaten (D4) von einer ersten Experteninformation (E1) über eine potenziell möglichen Sprungkraft des Athleten (A) erzeugt werden.

10. Verfahren nach Anspruch 8, **dadurch gekennzeichnet, dass** die im dritten Schritt (III) ermittelten vergleichbaren anderen Athletendaten (D12) eine Sprunggeschwindigkeitsinformation (F2) der anderen Athleten umfassen und die ebenfalls im dritten Schritt (III) ermittelten Leistungsdaten (D10) von einer Sprunggeschwindigkeitsinformation (Z2) des Athleten (A) mit den vergleichbaren anderen Athletendaten (D12) von einer Sprunggeschwindigkeitsinformation (F2) der anderen Athleten verglichen werden und Expertendaten (D4) von einer zweiten Experteninformation (E2) über eine potenziell mögliche Sprunggeschwindigkeit des Athleten (A) ermittelt werden.

11. Verfahren nach Anspruch 8, **dadurch gekennzeichnet, dass** die im dritten Schritt (III) ermittelten vergleichbaren anderen Athletendaten (D12) eine Sprungleistungsinformation (F3) der anderen Athleten umfassen, und die ebenfalls im dritten Schritt (III) ermittelten Leistungsdaten (D10) von einer Sprungleistungsinformation (Z4) des Athleten (A) mit den vergleichbaren anderen Athletendaten (D12) von einer Sprungleistungsinformation (F3) der anderen Athleten verglichen werden und Expertendaten (D4) von einer dritten Experteninformation (E3) über eine potenziell möglichen Sprungleistung des Athleten (A) ermittelt werden.

12. Verfahren nach Anspruch 8, **dadurch gekennzeichnet, dass** die im dritten Schritt (III) ermittelten vergleichbaren anderen Athletendaten (D12) eine Sprungkraftinformation (F4) über eine Differenz der Sprungkraft der beiden unteren Extremitäten der anderen Athleten umfassen und die ebenfalls im dritten Schritt (III) ermittelten Leistungsdaten (D10) von einer Sprungkraftinformation (Z3) des Athleten (A) mit den vergleichbaren anderen Athletendaten (D12) von einer Sprungkraftinformation (F4) über eine Differenz der Sprungkraft der beiden unteren Extremitäten der anderen Athleten verglichen werden und Expertendaten (D4) von einer vierten Experteninformation (E4) über eine Differenz der Sprungkraft der beiden unteren Extremitäten des Athleten (A) ermittelt werden.

13. Verfahren nach Anspruch 8, **dadurch gekennzeichnet, dass** die im dritten Schritt (III) ermittelten Leistungsdaten (D10) von einer Sprungkraftinformation (Z3) des Athleten (A) mit Leistungsdaten (D10) von einer Sprunggeschwindigkeitsinformation (Z2) des Athleten (A) in Beziehung gesetzt und Leistungsdaten (10) von einer Sprungkraft-Sprunggeschwindigkeitsinformation (Z6) des Athleten (A) gebildet werden; dass im dritten Schritt (III) biometrische Daten (D13) von einer maximal möglichen Sprungkraftinformation (B1) eines Athleten und biometrische Daten (D13) von einer maximal möglichen Sprunggeschwindigkeitsinformation (B2) eines Athleten bereitgestellt werden; dass die biometrischen Daten (D13) von einer maximal möglichen Sprungkraftinformation (B1) eines Athleten mit biometrischen Daten (D13) von einer maximal möglichen Sprunggeschwindigkeitsinformation (B2) eines Athleten in Beziehung gesetzt und biometrische Daten (13) von einer Sprungkraft-Sprunggeschwindigkeitsinformation (B4) eines Athleten gebildet werden; und dass die Leistungsdaten (10) von der Sprungkraft-Sprunggeschwindigkeitsinformation (Z6) des Athleten (A) mit den biometrischen Daten (D13) von der Sprungkraft-Sprunggeschwindigkeitsinformation (B4) eines Athleten verglichen werden und Expertendaten (D4) von einer fünften Experteninformation (E5) über eine Sprungkraft-Sprunggeschwindigkeit-Beziehung des Athleten (A) ermittelt.

14. Verfahren nach Anspruch 12, **dadurch gekennzeichnet, dass** im dritten Schritt (III) medizinische Daten (D14) über ein zukünftiges Verletzungsrisiko infolge einer Differenz der Sprungkraft der beiden unteren Extremitäten eines Athleten bereitgestellt werden; dass die Expertendaten (D4) der vierten Experteninformation (E4) über eine Differenz der Sprungkraft der beiden unteren Extremitäten des Athleten (A) mit den medizinischen Daten (D14) verglichen werden und Expertendaten (D4) von einer sechsten Experteninformation (E6) über ein zukünftiges Verletzungsrisiko des Athleten (A) ermittelt werden.

15. Verfahren nach einem der Ansprüche 8 bis 14, **dadurch gekennzeichnet, dass** im vierten Schritt (IV) Expertendaten (D4) als Experteninformation (E1 bis E6) auf einem ersten Ausgabemittel (AU5) der ersten Rechnereinrichtung (5) und/oder auf einem zweiten Ausgabemittel (AU6) der zweiten Rechnereinrichtung (6) ausgegeben werden.

16. Vorrichtung (10) zur Durchführung des Verfahrens nach einem der Ansprüche 1 bis 15, mit einer Messplattform (1), einer Datenverarbeitungsanlage (4) und einer ersten Rechnereinrichtung (5), **dadurch gekennzeichnet, dass** die Vorrichtung (10) auch eine Datenübertragungsvorrichtung (3) aufweist; dass die Datenverarbeitungsanlage (4) über die Datenübertragungsvorrichtung (3) Übungsdaten (D5) für Sprungübungen an die erste Rechnereinrichtung (5) sendet; dass die Messplattform (1) einen Messplattform-Prozessor (P1) aufweist, der derart konfiguriert ist, dass er für auf der Messplattform (1) ausgeführte Sprungübungen Messdaten (D1) erzeugt; dass die Messplattform (1) über die Datenübertragungsvorrichtung (3) die Messdaten (D1) an die Datenverarbeitungsanlage (4) sendet; dass die Datenverarbeitungsanlage (4) einen Haupt-Prozessor (P4) aufweist, der derart konfiguriert ist, dass er die Messdaten (D1) zu Leistungsdaten (D10) auswertet und dass er für die Leistungsdaten (D10) Expertendaten (D4) ermittelt; und dass die Datenübertragungsvorrichtung (3) über die Datenübertragungsvorrichtung (3) die Leistungsdaten (D10) und die Expertendaten (D4) an mindestens eine der Folgenden, der ersten Rechnereinrichtung (5) und einer zweiten Rechnereinrichtung (6) sendet.
